# EUROPEAN PATENT APPLICATION

(11) **EP 1 826 268 A1**
(43) Date of publication of application: **29.08.2007**
(21) Application number: 04766993.2
(22) Date of filing: 16.09.2004
(51) Int. Cl.: C12N 15/00, A01K 67/027, C12N 5/06, C12N 5/10, G01N 33/50, C07K 14/715

(54) **CCR6- DEFICIENT MICE OR CELLS AND MODULATION OF INFLAMMATORY DISEASES**

(71) Applicant: Consejo Superior de Investigaciones Cientificas, 28006 Madrid (ES); Pharmacia Spain S.A., 08190 Sant Cugat de Valles (Barcelona) (ES)
(72) Inventor: VARONA, Rosa Centro Nacional de Biotecnologia, E-28049 Madrid (ES); MARTINEZ-ALONSO, Carlos Ctr. Nacional de Biotecn., E-28049 Madrid (ES); MARQUEZ, Gabriel Centro Nacional de Biotecnol., E-28049 Madrid (ES)
(74) Representative: Elzaburu Marquez, Alberto
(86) International application number: PCT/ES2004/070070
(87) International publication number: WO 2006/056623

(57) **Abstract**

The present invention generally relates to the generation of a knockout mouse for CCR6 and particularly, but not exclusively, to the identification of CCR6 as a target for drugs that reduce inflammatory immunological responses. More specifically, the invention relates to, but in not limited to, non-human animals and cells deficient in the CCR6 receptor, to the use of CCR6 as a target for drugs and to the use of CCR6 inhibitors for graft versus host disease, cutaneous inflammations such as allergic contact dermatitis and intestinal inflammations such as ulcerative colitis and Crohn's disease.

## Description

### FIELD OF THE INVENTION

The present invention relates generally to the generation of a CCR6 knockout mouse (genetically deprived of CCR6), and particularly, but not by way of limitation, to the identification of CCR6 as a target for drugs that reduce inflammatory, immunological responses. The invention relates more particularly, but not by way of limitation, to cells and non-human animals deficient for the CCR6 receptor, the use of CCR6 as a target for drugs, and the therapeutic use of inhibitors of CCR6 to treat graft-versus-host disease, cutaneous inflammations such as allergic contact dermatitis, and intestinal inflammations such as ulcerative colitis and Crohn's disease.

### BACKGROUND

The information provided below is not admitted to be prior art to the present invention, but is provided solely to assist the understanding of the reader.

Leukocyte traffic is the hallmark of the immune response. Controlled cell movements allow the precise interactions necessary to bring different leukocytes into physical contact. The possibility of rare T and B lymphocytes encountering their specific antigen is maximized by their recirculation through secondary lymphoid organs, in which antigens are displayed to them by antigen-presenting cells. Of these, dendritic cells (DCs) are an important heterogeneous population of antigen-presenting cells that can induce, sustain, and regulate immune responses (J. Banchereau et al. 2000. Immunobiology of Dendritic Cells. Annu. Rev. Immunol. 18:767-811). Once activated, effector T cells are able to migrate to tissues in which pathogens have been detected. Eventually, memory T cells constitute a surveillance system that ensures tissue protection in case of a new antigen challenge. Chemokines are a family of proteins that control leukocyte trafficking by binding to specific seven-transmembrane receptors [P.M Murphy, M. Baggiolini, I.F. Charo, C.A. Hebert, R. Horuk, K. Matsushima, L.H. Miller, J. J. Oppenheim. And C.A. Power, International Union of Pharmacology. XXII. Nomenclature for chemokine receptors. Pharmacol. Rev. 2000. 52: 145-176; A. Zlotnik and O. Yoshie, Chemokines: a new classification system and their role in immunity. Immunity 2000, 12: 121-127].

In recent years, several reports have pointed to chemokines as important factors in the regulation of leukocyte trafficking for lymphoid organ architecture, and in physiological and pathological situations (R. Forster et al., 1996. A putative chemokine receptor, BLR1, directs B cell migration to defined lymphoid organs and specific anatomic compartments of the spleen. Cell. 87:1037-1047; F. Sallusto, A. Lanzavecchia and C. R. Mackay, 1998, Chemokines and chemokine receptors in T-cell priming and Th1/Th2-mediated responses; Immunol. Today. 19:568-574; F. Sallusto, D. Lenig, R. Forster, M. Lipp and A. Lanzavecchia, 1999, Two subsets of memory T lymphocytes with distinct homing potentials and effector functions. Nature. 401:708-712; R. Forster et al., 1999, CCR7 coordinates the primary immune response by establishing functional microenvironments in secondary lymphoid organs, Cell, 99:23-33; J. G. Cyster, 1999, Chemokines and cell migration in secondary lymphoid organs, Science, 286:2098-2102; J. J. Campbell and E. C. Butcher, 2000, Chemokines in tissue-specific and microenvironment-specific lymphocyte homing, Curr. Opin. Immunol., 12:336-341; B. Moser and P. Loetscher, Lymphocyte traffic control by chemokines, Nat. Immunol., 2001, 2: 123-128; K. M. Ansel and J. G. Cyster, Chemokines in lymphopoiesis and lymphoid organ development, Curr. Opin. Immunol., 2001, 13: 172-179; F. Sallusto, C. R. Mackay, and A. Lanzavecchia, The role of chemokine receptors in primary, effector, and memory immune responses, Annu. Rev. Immunol., 2000, 18: 593-620; C. Gerard and B. J. Rollins, Chemokines and disease, Nat. Immunol., 2001, 2: 108-115).

CCR6 is a β-chemokine-specific receptor for CCL20 (MIP-3aα/LARC/Exodus) (M. Baba et al., 1997, Identification of CCR6, the specific receptor for a novel lymphocyte-directed CC chemokine LARC, J. Biol. Chem. 272:14893-14898; F. Liao et al., 1997, STRL22 is a receptor for the CC chemokine MIP-3α, Biochem. Biophys. Res. Commun. 236:212-217; C. A. Power et al., 1997, Cloning and characterization of a specific receptor for the novel CC chemokine MIP-3α from lung dendritic cells, J. Exp. Med. 186:825-835; D. R. Greaves et al., 1997, CCR6, a CC chemokine receptor that interacts with macrophage inflammatory protein 3α and is highly expressed in human dendritic cells, J. Exp. Med. 186:837-844; D.L. Rossi, A. P. Vicari, B. K. Franz, T. K. McClanahan and A. Zlotnik, 1997, Identification through bioinformatics of two new macrophage proinflammatory human chemokines: MIP-3α and MIP-3β, J. Immunol. 158: 1033-1036; R. Hromas et al., 1997, Cloning and characterization of exodus, a novel β-chemokine. Blood. 89:3315-3322). In addition to having only one highly specific ligand, other features make CCR6 an interesting receptor. Human CCR6 is expressed in immature DCs derived in vitro from CD34+ precursors and is downregulated as DCs mature (M. C. Dieu et al. 1998, Selective recruitment of immature and mature dendritic cells by distinct chemokines expressed in different anatomic sites, J Exp. Med. 188:373-386; L. Carramolino et al., 1999, Down-regulation of the β-chemokine receptor CCR6 in dendritic cells mediated by TNF-α and IL-4, J. Leukoc. Biol. 66:837-844.); it is also expressed in memory T cells, CLA+ cells and B cells (F. Liao et al., 1999, CC-chemokine receptor 6 is expressed on diverse memory subsets of T cells and determines responsiveness to macrophage inflammatory protein 3α, J. Immunol. 162: 186-194). Similar CCR6 expression patterns have been reported in the mouse, in which CCR6 is expressed in the myeloid but not in the lymphoid DC subpopulation (R. Varona et al., 1998, Molecular cloning, functional characterization and mRNA expression analysis of the murine chemokine receptor CCR6 and its specific ligand MIP-3α. FEBS Lett. 440: 188-194; a. Iwasaki and B. L. Kelsall, 2000, Localization of distinct Peyer's patch dendritic cell subsets and their recruitment by chemokines macrophage inflammatory protein (MIP)-3α, MIP-3β, and secondary lymphoid organ chemokine, J. Exp. Med. 191:1381-1394), B cells, and CD4+ T cells (R. Varona et al., 1998. Molecular cloning, functional characterization and mRNA expression analysis of the murine chemokine receptor CCR6 and its specific ligand MIP-3α, FEBS Lett. 440: 188-194).

CCL20 is known to interact only with CCR6 in both the human and murine systems. Human CCL20 triggers adhesion of memory CD4+ T cells to ICAM-1 (J. J. Campbell et al., 1998. Chemokines and the arrest of lymphocytes rolling under flow conditions, Science, 279: 381-384) and is expressed in epithelial crypts of inflamed tonsils (M. C. Dieu et al., 1998. Selective recruitment of immature and mature dendritic cells by distinct chemokines expressed in different anatomic sites. J Exp. Med. 188:373-386), epithelial cells from appendix (Y. Tanaka et al., 1999. Selective expression of liver and activation-regulated chemokine (LARC) in intestinal epithelium in mice and humans, Eur. J. Immunol. 29:633-642.), as well as keratinocytes and venular endothelial cells from non pathological skin (A. S. Charbonnier et al., 1999. Macrophage inflammatory protein 3a is involved in the constitutive trafficking of epidermal Langerhans cells, J. Exp. Med. 190:1755-1768) and psoriatic skin (B. Homey et al., 2000, Up-regulation of macrophage inflammatory protein-3α/CCL20 and CC chemokine receptor 6 in psoriasis, J. Immunol. 164:6621-6632; M. C. Dieu-Nosjean et al., 2000, Macrophage inflammatory protein 3α is expressed at inflamed epithelial surfaces and is the most potent chemokine known in attracting Langerhans cell precursors, J. Exp. Med. 192:705-717).

Murine CCL20 (mCCL20) was also reported to be expressed in epithelial cells from intestinal tissue (Y. Tanaka et al., 1999, Selective expression of liver and activation-regulated chemokine (LARC) in intestinal epithelium in mice and humans., Eur.J. Immunol. 29:633-642). Interestingly, in Peyer's patches (PPs), mCCL20 is expressed only by the follicle-associated epithelium (FAE) overlying the subepithelial dome (SED), where CCR6-expressing cells accumulate (A. Iwasaki and B. L. Kelsall, 2000, Localization of distinct Peyer's patch dendritic cell subsets and their recruitment by chemokines macrophage inflammatory protein (MIP)-3α, MIP-3β, and secondary lymphoid organ chemokine, J. Exp. Med. 191:1381-1394).

Taken together, these data suggest a role for CCR6 and its ligand as regulators of the migration and recruitment of antigen-presenting and immunocompetent cells during inflammatory and immunological responses.

*Role of CCR6 in contact hypersensitivity (CHS) and delayed-type hypersensitivity (DTH) models of inflammation.* CCR6-/- mice (CCR6 knockouts) were generated to investigate the role of CCR6 in vivo. Analysis of CCR6 knockout mice revealed defects in leukocyte homing to the intestinal mucosa as evidenced by their underdeveloped Peyer's patches, in which clear alterations in the positioning of myeloid CD11b+ CD11c+ DCs were observed. These animals also showed increased numbers of intraepithelial lymphocyte (IEL) subpopulations. Allergic contact dermatitis, also known as contact hypersensitivity, is a T cell-dependent skin disease with the kinetics of a delayed-type hypersensitivity response (S. Grabbe and T. Schwarz, 1998, Immunoregulatory mechanisms involved in the elicitation of allergic contact hypersensitivity, Immunol. Today 19:37-44). This disorder is rarely life-threatening, but the costs to society of occupation-related allergic contact dermatitis are high (R. L. Riestchel, 1995, Human and economic impact of allergic contact dermatitis and the role of patch testing, J. Acad. Dermatol. 33:812-815). In this disease, the antigen is introduced epicutaneously through intact skin. The sensitizing antigens are typically unstable reactive molecules that can form complexes with host proteins. Potent contact-sensitizing antigens induce a dose-dependent cutaneous irritation that is independent of their antigenicity. The altered responses of CCR6-/- mice in contact hypersensitivity (CHS) and delayed-type hypersensitivity (DTH) models of inflammation suggest a critical role for CCR6 in the activation and/or migration of CD4+ T-cell subsets that home into skin.

*Role of CCR6 in the development of inflammatory bowel disease.* The intestine is bathed by a large variety of foreign antigens, which normally elicit an appropriate immune response. Defects in the mechanisms that control the reaction to these antigens are thought to provoke the excessive immunological response that may lead to tissue destruction and intestinal inflammatory disease (C. Nagler-Anderson, Man the barrier! Strategic defences in the intestinal mucosa, Nat. Rev. Immunol. 2001. 1: 59-67).

Among these pathologies, human inflammatory bowel disease (IBD) is a chronic, relapsing and remitting inflammatory condition, clinically characterized by two phenotypes, ulcerative colitis and Crohn's disease, that affect the colon and/or the distal small intestine (R. S. Blumberg, L. J. Saubermann and W. Strober, Animal models of mucosal inflammation and their relation to human inflammatory bowel disease, Curr. Opin. Immunol., 1999, 11: 648-656).

Chemokine overexpression has been observed in patients with ulcerative colitis or Crohn's disease (L. Mazzucchelli, C. Hauser, K. Zgraggen, H. Wagner, M. Hess, J. A. Laissue and C. Mueller, Expression of interleukin-8 gene in inflammatory bowel disease is related to the histological grade of active inflammation, Am. J. Pathol. 1994, 144:997-1007; M. C. Grimm, S. K. Elsbury, P. Pavli and W. F. Doe, Enhanced expression and production of monocyte chemoattractant protein-1 in inflammatory bowel disease mucosa. J. Leukoc. Biol. 1996. 59: 804-812; S. Keates, A. C. Keates, E. Mizoguchi, A. Shan and C. P. Kelly, Enterocytes are the primary source of the chemokine ENA-78 in normal colon and ulcerative colitis, Am. J. Physiol. 1997, 273:G75-G82; J. Wedemeyer, A. Lorentz, M. Goke, P. N. Meier, P. Flemming, C. A. Dahinden, M. P. Manns and S. C. Bischoff, Enhanced production of monocyte chemotactic protein 3 in inflammatory bowel disease mucosa, Gut 1999. 44: 629-635; L. Mazzucchelli, C. Hauser, K. Zgraggen, H. E. Wagner, M. W. Hess, J. A. Laissue and C. Mueller, Differential in situ expression of the genes encoding the chemokines MCP-1 and RANTES in human inflammatory bowel disease, J. Pathol. 1996. 178: 201-206).

CCL20 expression in epithelia from tonsils [Dieu-Nosjean et al., Selective recruitment of immature and mature dendritic cells by distinct chemokines expressed in different anatomic sites, J. Exp. Med. 1998. 188: 373-386], appendix, colon and other intestinal tissue [Y. Tanaka et al., 1999. Selective expression of liver and activation-regulated chemokine (LARC) in intestinal epithelium in mice and humans, Eur. J. Immunol. 29: 633-642; A. Izadpanah, M. B. Dwinell, L. Eckmann, N. M. Varki and M. F. Kagnoff, Regulated MIP-3α/CCL20 production by human intestinal epithelium: mechanism for modulating mucosal immunity, Am. J. Physiol. Gastrointest. Liver Physiol. 2001, 280: G710-G719], Peyer's patches [A. Iwasaki and B. L. Kelsall, Localization of distinct Peyer's patch dendritic cell subsets and their recruitment by chemokines macrophage inflammatory protein (MIP)-3α, MIP-3β, and secondary lymphoid organ chemokine, J. Exp. Med. 2000, 191:1381-1394], as well as normal [ A. S. Charbonnier et al. 1999, Macrophage inflammatory protein 3α is involved in the constitutive trafficking of epidermal Langerhans cells, J. Exp. Med. 190:1755-1768) and psoriatic skin (B. Homey, et al., 2000, Up-regulation of macrophage inflammatory protein-3α/CCL20 and CC chemokine receptor 6 in psoriasis, J. Immunol. 164:6621-6632; M. C. Dieu-Nosjean et al., 2000, Macrophage inflammatory protein 3α is expressed at inflamed epithelial surfaces and is the most potent chemokine known in attracting Langerhans cell precursors, J. Exp. Med. 192:705-717) supports the hypothesis that this chemokine has an important function in the interface between the organism and the environment.

A recent study of CCR6-deficient mice demonstrated CCR6/CCL20 involvement in the control of leukocyte homeostasis in the gut (R. Varona, R. Villares, L. Carramolino, I. I. Goya, A. A. Zaballos, J. Gutierrez, M. Torres, A. C. Martinez and G. Marquez, CCR6-deficient mice have impaired leukocyte homeostasis and altered contact hypersensitivity and delayed-type hypersensitivity responses. J. Clin. Invest. 2001. 107: R37-R45). In CCR6-deficient mice, CD11 b+CD11c+ myeloid DC are clearly decreased in the subepithelial dome of PP, since these cells are displaced mainly to the interfollicular region (R. Varona, R. Villares, L. Carramolino, I. I. Goya, A. A. Zaballos, J. Gutierrez, M. Torres, A. C. Martinez, and G. Marquez, CCR6-deficient mice have impaired leukocyte homeostasis and altered contact hypersensitivity and delayed-type hypersensitivity responses. J. Clin. Invest. 2001. 107: R37-R45). CCR6-deficient mice also have increased numbers of T cell subpopulations within the intestinal mucosa (R. Varona, R. Villares, L. Carramolino, I. I. Goya, A. A. Zaballos, J. Gutierrez, M. Torres, A. C. Martinez, and G. Marquez, CCR6-deficient mice have impaired leukocyte homeostasis and altered contact hypersensitivity and delayed-type hypersensitivity responses. J. Clin. Invest. 2001. 107: R37-R45).

The immune response has been now studied in two IBD models in CCR6-deficient mice. The IBD models are mediated by the administration of dextran sodium sulfate (DSS) or 2,4,6-trinitrobenzene sulfonic acid (TNBS). The obtained data show that the absence of CCR6 results in less severe intestinal pathology in DSS-treated mice, with no ulcerations observed in colon. CCR6 deficiency alters leukocyte homeostasis and the cytokine environment in the intestinal mucosa. These changes are sufficient to confer susceptibility to TNBS-mediated intestinal inflammation in the otherwise resistant C57B1/6J mouse strain. Taken together, our data suggest that the CCR6/CCL20 axis has a critical, non-redundant role in the control of immune responses in the intestine.

*CCR6 regulation of CD4+ T cell-mediated acute graft-versus-host disease responses.* Graft-versus-host disease (GvHD) is a common, debilitating complication of allogeneic bone marrow transplantation (V. T. Ho & R. J. Soiffer, The history and future of T-cell depletion as graft-versus-host disease prophylaxis for allogeneic hematopoietic stem cell transplantation. Blood 98, 3192-3204 (2001)). The disease is a multistep process involving lymphocyte activation and proliferation and target cell killing. Donor T cells recognize the major histocompatibility complex (MHC) and associated peptides on host-derived antigen-presenting cells. This leads to cell activation and differentiation into various effector and memory T cells with selective tropism [F. Sallusto, D. Lenig, R. Forster, M. Lipp and A. Lanzavecchia, Two subsets of memory T lymphocytes with distinct homing potentials and effector functions, Nature 401,708-712 (1999); J. H. Antin, Acute graft-versus-host disease: inflammation run amok? J. Clin. Invest. 107, 1497-1498 (2001)]. In acute GvHD, activated T cells infiltrate mainly the skin, liver, and gastrointestinal tract, where they initiate a Th1-mediated immune response. Interferon-γ (IFN-γ) collaborates in recruitment of other T cell subsets, monocytes, and macrophages that further damage affected tissues (W. Krenger and J. L. Ferrara, Graft-versus-host disease and the Th1/Th2 paradigm, Immunol. Res. 15, 50-73 (1996); W. D. Shlomchik et al., Prevention of graft versus host disease by inactivation of host antigen-presenting cells, Science 285, 412-415 (1999)). Recent data showing that neutralization of TNF-α and IL-1 prevents acute GvHD confirm the central role of inflammatory cytokines in this disease [T. Teshima et al., Acute graft-versus-host disease does not require alloantigen expression on host epithelium, Nat. Med. 8: 575-581 (2002)].

The adhesion and chemoattractant receptors expressed by effector/memory T cells interact with the adhesion molecules and chemokines expressed by target tissues, controlling T cell homing specificity [E. J. Kunkel and E. C. Butcher, Chemokines and the tissue-specific migration of lymphocytes, Immunity 16, 1-4 (2002)]. The chemokine CCL20 and its receptor CCR6 have a central role in chemoattracting specific T, B, and dendritic cell subsets to the peripheral tissues and lymphoid organs associated to epithelial surfaces [E. Schutyser, S. Struyf, and J. Van Damme, The CC chemokine CCL20 and its receptor CCR6, Cytokine Growth Factor Rev. 14, 409-426 (2003)]. CCR6 expression is restricted to the CLA+, α₄β₇+, or CLA-α₄β₇- cell subsets of the effector/memory T cell pool, which home to skin, gut or other non-lymphoid peripheral tissues, respectively [F. Liao et al., CC-chemokine receptor 6 is expressed on diverse memory subsets of T cells and determines responsiveness to macrophage inflammatory protein 3α, J. Immunol. 162: 186-194 (1999)]. This suggests that CCR6 is induced on T cells that are activated in diverse environments.

It has been shown now that mucosal immunity is impaired in CCR6-deficient mice and that these animals have altered immune responses to cutaneous and intestinal inflammations [R. Varona et al., CCR6-deficient mice have impaired leukocyte homeostasis and altered contact hypersensitivity and delayed-type hypersensitivity responses, J. Clin. Invest. 107: R37-R45 (2001); R. Varona, V. Cadenas, J. Flores, A. C. Martinez and G. Marquez, CCR6 has a non-redundant role in the development of inflammatory bowel disease, Eur. J. Immunol. 33: 2937-2946 (2003)]. CCL20 is expressed constitutively by keratinocytes and venular endothelial cells from normal skin, as well as by mucosa-associated tissues; inflammatory stimuli upregulate these basal levels [E. Schutyser, S. Struyf and J. Van Damme, The CC chemokine CCL20 and its receptor CCR6, Cytokine Growth Factor Rev. 14, 409-426 (2003)]. The data available indicate that CCL20 acts through CCR6 to recruit differentiated memory cells to inflammation sites in skin and mucosa.

The role of CCR6 in acute GvHD development in MHC-mismatched recipients was than analyzed. Disease onset was delayed and the mortality ratio decreased in MHC class 11-mismatched recipients of CD4+ T cells from CCR6-deficient compared to wild type (WT) donors. Conversely, CD8+ T cells from both CCR6-deficient and WT donors provoked similar GvHD symptoms in MHC class 1-mismatched recipients. CCR6 was also critical for GvHD development across minor histocompatibility complex (mhc)-mismatched barriers. Onset of skin lesions and intestinal inflammation was delayed and symptoms were milder in MHC-matched/mhc-mismatched recipients of naive CD4+CD45RB^{high} T cells from CCR6-deficient donors. Significant delay was observed in CD45+ and CD4+ cell infiltration to skin and intestine in recipients of naive T cells from CCR6-deficient donors. We found lower IFN-γ and IL-10 levels in skin and gut of these mice. Moreover, the levels of the chemokines that control activated T cell homing to skin and gut were also decreased. These data suggest that by facilitating alloreactive effector/memory CD4+ T cell recruitment to target tissues, CCR6 plays a major role in GvHD development. Transferred CD4⁺CD45RB^{low} memory/regulatory T cells do not provoke GvHD in recipient mice and are able to suppress GvHD caused by CD4⁺CD45RB^{high} naïve T cells in cotransfer experiments (M.P. Schon, M. Detmar and C M. Parker, 1997, Murine psoriasis-like disorder induced by naive CD4+ T cells, Nat. Med. 3: 183-188; P. J. Morrissey, K. Charrier, S. Braddy, D. Liggitt and J.D. Watson, 1993, CD4+ T cells that express high levels of CD45RB induce wasting disease when transferred into congenic severe combined immunodeficient mice. Disease development is prevented by cotransfer of purified CD4+ T cells. J Exp Med 178: 237-244). These studies showed that CD4⁺CD45RB^{low} memory/regulatory T cells from CCR6-deficient mice failed to suppress GvHD produced by WT naïve T cell transfer, indicating a critical role for CCR6 in the regulatory function of this T cell subset.

Other objects and advantages will become apparent from the following disclosure.

### SUMMARY OF THE INVENTION

To study the role of CCR6 in several inflammation diseases, the inventors have used gene targeting by homologous recombination to create mutant mice that lack CCR6. The present invention provides a transgenic, non-human, knockout animal whose genome comprises an introduced null mutation in at least one CCR6 allele.

An aspect of the present invention provides a mouse cell line designated CCR6KOES192, deposited with the Deutsche Sammlung von Mikroorganismen und Zellkulturen GmbH (DSM) under accession number DSM ACC2636. A further aspect provides a mouse, or part thereof, comprising cells grown from cells of DSM ACC2636.

An aspect of the present invention provides a mammalian cell having a deletion in at least one CCR6 allele and having the characteristics of the mouse cell line designated CCR6KOES192, deposited with the DSM under accession number DSM ACC2636. A further aspect provides a mouse, or part thereof, comprising cells having the characteristics of the mouse cell line designated CCR6KOES192, deposited with the DSM under accession number DSM ACC2636.

An aspect of the present invention provides a transgenic, non-human, knockout animal whose genome comprises an introduced null mutation in at least one CCR6 allele, wherein said mutation has the characteristics of the cell line designated CCR6KOES192, deposited with the DSM under accession number DSM ACC2636.

An aspect of the present invention provides that the knockout animal is a rodent.

A further aspect provides that the knockout animal is a mouse.

The present invention provides a knockout mouse whose genome comprises an introduced null mutation in both CCR6 alleles.

The present invention provides a method of making a transgenic knockout mouse having a disrupted CCR6 gene comprising: deleting the first exon of the CCR6 gene including the initiation codon by homologous recombination in a mouse embryonic stem cell; aggregating a clump of said stem cells with morulae and developing the corresponding blastocysts *in vitro;* transplanting said blastocyst into a pseudopregnant mouse; allowing said blastocyst to develop into a chimeric mouse; breeding said chimeric mouse to produce offspring; and screening said offspring to identify a homozygous transgenic knockout mouse whose genome comprises deletion of the first exon of both alleles of the CCR6 gene.

The present invention provides a method to assay for a test compound that moderates CCR6-dependent GvHD comprising: an assay for binding of the test compound to CCR6, determining whether the test compound inhibits CCR6, providing CD4⁺ T cells from a WT mouse; providing a CCR6 WT, MHC(II)-mismatched mouse; providing a test compound; transferring said T cells to said WT mouse in the presence and absence of said test compound; and comparing a GvHD response in the presence and absence of said test compound.

An aspect of the present invention provides a method to assay for a compound that moderates CCR6-dependent GvHD, wherein the GvHD response is selected from the group consisting of % survival at inflection day post transfer, recipient weight at inflection day, ear inflammation, skin lesions, diarrhea, spleen index, and CD4⁺ T cell expansion.

The present invention provides a method to assay for a compound that moderates CCR6-dependent, inflammatory bowel disease comprising: providing a C57BL/6J WT mouse; rendering said mouse as DSS-type IBD models; administering a test compound to said mouse; and comparing an IBD response of said mouse in the presence and absence of said test compound.

An aspect of the present invention provides a method to assay for a compound that moderates CCR6-dependent, inflammatory bowel disease, wherein the IBD response is selected from the group showing intestinal inflammation and appropriate colonic mucosa Th1 and Th2 cytokine mRNA levels.

The present invention provides a method to assay for a compound that moderates CCR6-dependent, inflammatory bowel disease comprising: providing a C57BL/6J WT mouse; administering trinitrobenzenesulfonic acid to said mouse; administering a test compound to said mouse; and comparing an IBD response of said mouse in the presence and absence of said test compound.

An aspect of the present invention provides a method to assay for a compound that moderates CCR6-dependent, TNBS-type inflammatory bowel disease, wherein the IBD response is selected from the group showing intestinal inflammation and appropriate colonic mucosa Th1 and Th2 cytokine mRNA levels.

The present invention provides a method to assay for a compound that moderates CCR6-dependent, contact hypersensitivity inflammation comprising: providing a C57BL/6J WT mouse; sensitizing said mouse with DNFB; challenging said mouse with DNFB; administering a test compound to said mouse; and comparing a contact hypersensitivity inflammation response of said mouse in the presence and absence of said test compound.

An aspect of the present invention provides a method to assay for a compound that moderates CCR6-dependent, DNFB-type contact hypersensitivity inflammation wherein said contact hypersensitivity inflammation response is ear inflammation.

Still other objects and advantages of the present invention will become readily apparent by those skilled in the art from the following detailed description, wherein it is shown and described preferred embodiments of the invention, simply by way of illustration of the best mode contemplated of carrying out the invention. As will be realized the invention is capable of other and different embodiments, and its several details are capable of modifications in various obvious respects, without departing from the invention. Accordingly, the description is to be regarded as illustrative in nature and not as restrictive.

### BRIEF DESCRIPTION OF DRAWINGS

The invention is best understood from the following detailed description when read in connection with the accompanying drawing. It is emphasized that, according to common practice, the various features of the drawing are not to scale. On the contrary, the dimensions of the various features are arbitrarily expanded or reduced for clarity. Included in the drawing are the following figures:
Figure 1(a) depicts a partial restriction map of the CCR6 WT (wild type) locus, the targeting vector used, and the resulting genetic modification of the CCR6 gene.
Figure 1(b) depicts a representative Southern blot analysis of BamH1-digested tail DNA from WT (+/+), heterozygous (+/-), and homozygous (-/-) CCR6 mice;
Figure 1(c) depicts a representative Northern blot analysis of CCR6 mRNA expressed in thymus and spleen from CCR6 WT (+/+) and knockout (-/-) mice;
Figure 2(a) shows altered ear inflammation response of CCR6-/- mice to DNFB-induced contact hypersensitivity inflammation;
Figure 2(b) shows that DNFB-induced contact hypersensitivity inflammation is not a function of differences in cell number in inguinal, axial and brachial lymph nodes of untreated (white bars) or treated (black bars) animals;
Figure 2(c) shows similar WT and CCR6-/- lymphocyte proliferation in response to specific and nonspecific stimuli;
Figure 3(a) shows a delayed-type hypersensitivity (DTH) response of CCR6 knockout and WT mice;
Figure 3(b) shows CCR6 knockout and WT, *in vitro* mixed lymphocyte reaction (MLR) to allogeneic BALB/c splenocytes;
Figure 3(c) shows that adoptive transfer of sensitized CCR6+/+ CD4+ T cells to CCR6-/- mice restores their ability to produce a DTH response;
Figure 4(a) shows that CCR6 deficiency protects against DSS-mediated colitis weight loss;
Figure 4(b) shows that CCR6 deficiency protects against DSS-mediated colitis intestinal inflammation;
Figure 4(c) shows altered cytokine response to DSS treatment in CCR6-deficient mice (white bars) compared to WT mice (black bars);
Figure 5(a) shows real-time quantitative analyses by reverse transcriptase - polymerase chain reaction (RT-PCR) of CD4 and CD8 transcripts in the colon of PBS- or TNBS-treated C57BL/6J WT or CCR6-deficient mice;
Figure 5(b) shows real-time quantitative RT-PCR analysis of cytokine and CD4 transcripts in the colon of PBS- or TNBS-treated BALB/c mice;
Figure 5(c) shows real-time quantitative RT-PCR analysis of cytokine transcripts in the colon of PBS- or TNBS-treated C57BL/6J WT or CCR6-deficient mice;
Figure 6(a) shows TNBS-induced colitis in BALB/c mice;
Figure 6(b) shows C57BL/6J WT mice are resistant to TNBS-induced colitis;
Figure 6(c) shows TNBS-induced colitis in C57BL/6J CCR6-deficient mice;
Figure 7 shows that the transfer of CD4⁺ T cells from CCR6-deficient mice to MHC class II-mismatched recipients (squares) results in milder GvHD compared to recipients of WT CD4⁺ T cells (circles);
Figure 8 shows that transfer of naive CD4⁺ T cells from CCR6-deficient mice to MHC-matched/mhc-mismatched recipients delays GvHD onset and reduces severity of symptoms, and that cotransfer of WT naïve CD4+ and CCR6-deficient regulatory T cells does not prevent or moderate the symptoms;
Figure 9 shows *that in vivo* homing of CD4⁺ T cells to secondary lymphoid organs and on-site proliferation is CCR6-independent;
Figure 10 shows that long-term expansion of CD4⁺ T cells is reduced in recipients of CCR6-deficient donor cells;
Figure 11 shows delayed CD4⁺ T cell homing to skin and small intestine of recipients of cells from CCR6-deficient donors; and
Figure 12 shows that recipients of CD4⁺ naive T cells from CCR6-deficient donors have lower and delayed expression of pro-inflammatory cytokines and chemokines in GvHD target organs.

It is to be noted, however, that the appended drawings illustrate only typical embodiments of this invention and are therefore not to be considered limiting of its scope, for the invention may admit to other equally effective embodiments.

### DETAILED DESCRIPTION OF A PREFERRED EMBODIMENT

### Definitions:

CHS and DHT studies used mice were 8- to 16-week-old 129 SvJ X C57BL/6 F3 or F4 CCR6-/- knockout mice. For DSS- and TNBS-mediated intestinal inflammation studies, as well as for the GvHD models, we used mice that had been backcrossed onto a C57BL/6 background for 8 generations.

Wild type (WT) mice, for the CHS and DHT studies, were 8- to 16-week-old 129 SvJ X C57BL/6 F3 or F4 CCR6+/+ mice. For the DSS- and TNBS-mediated intestinal inflammation studies, as well as for the GvHD models, mice on a C57BL/6 background were used.

A test compound is any ion, molecule, compound, or complex that binds to a specified macromolecule. Specifically for the purposes of the present invention, a test compound is that agent that binds to a CCR6 receptor. A "test compound" is any ion, molecule, compound, or complex that is being tested for its ability to bind to a specified macromolecule, preferably, a CCR6 receptor. A moderating test compound is any test compound, the binding of which affects the activity of the CCR6 in any of its roles in signal transduction. Moderation includes activation, inhibition, potentiation, agonism, and antagonism.

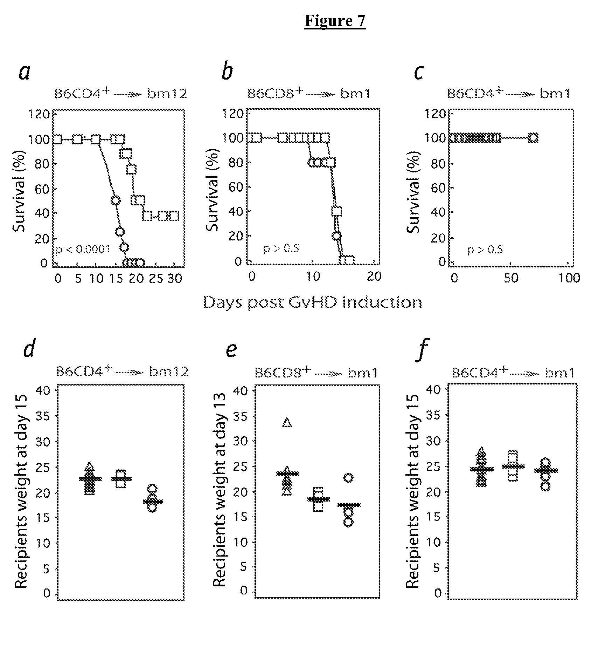

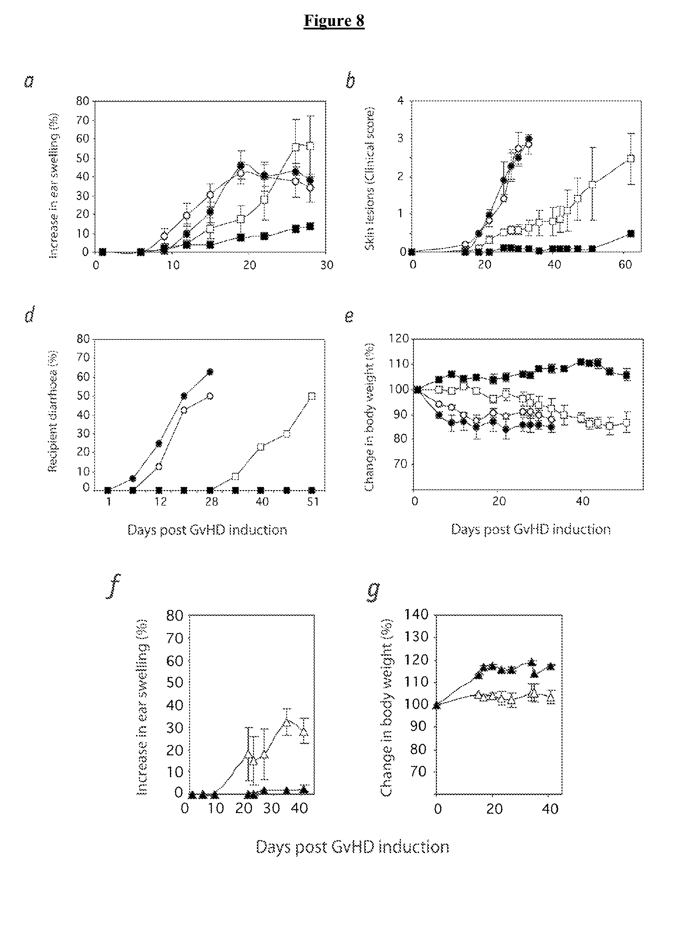

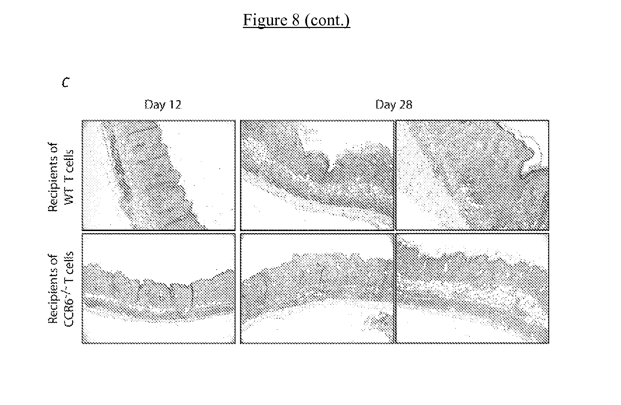

GvHD response may be determined as the % survival at a specified time post induction of GvHD. A preferred day is day 15 post-induction. As shown in Figure 7a, the curve of survival is decreasing sigmoid as a function of time post induction. A preferred day to determine % survival is that day correlating to the inflection point in the survival curve. Alternative measures of GvHD response include, but are not limited to, recipient weight at inflection day, Fig 7d; ear inflammation, Fig 8a, skin lesions, diarrhea, spleen index, and CD4⁺ T cell expansion.

Reference is made to the figures to illustrate selected embodiments and preferred modes of carrying out the invention. It is to be understood that the invention is not hereby limited to those aspects depicted in the figures.

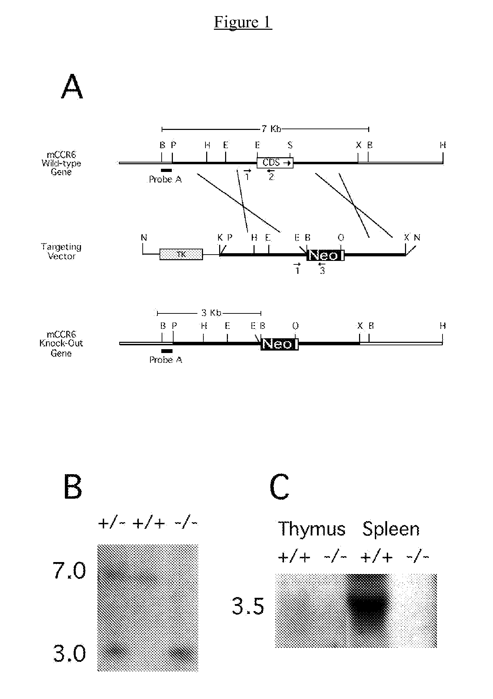

Gene Targeting. Gene targeting was performed according to established methods known to persons of skill. A 7-kb BamHI DNA fragment containing the CCR6 gene was subcloned from a phage P₁ mouse genomic library (Genome Systems Inc., St. Louis, Missouri, USA), as described [R. Varona, et al., Molecular cloning, functional characterization and mRNA expression analysis of the murine chemokine receptor CCR6 and its specific ligand MIP-3α. FEBS Lett. 188 (1998)]. A 2.7kb *Pst*I*-Eco*RI and a 2.7-kb *Sma*I*-Xba*I fragment from the CCR6 gene 5' and 3' regions, respectively, were then subcloned at either end of a neomycin resistance gene, under the control of the phosphoglycerate kinase promoter. The herpes simplex thymidine kinase gene was fused at the 5' end of the cloned CCR6 sequences in the replacement targeting construct, which was then linearized by NotI digestion and electroporated into the 129 SvJ R1 embryonic stem (ES) cell line. Ganciclovir and G418-resistant clones were selected, and 10 micrograms of genomic DNA from each clone was BamHI digested, subjected to electrophoresis, and blotted onto Hybond-N+ membranes (Amersham Pharmacia Biotech, Piscataway, New Jersey, USA). High-stringency hybridizations with CCR6-specific ³²P-labeled probes were performed in Rapid-Hyb buffer (Amersham Pharmacia Biotech). Probe A was derived from a region upstream of the 5' homology region (Figure 1).

The targeting strategy is presented schematically in Figure 1 (a), which shows the CCR6 WT locus with partial restriction map. The coding sequence (CDS), neomycin resistance gene (Neo), and thymidine kinase gene (TK) are shown as open, filled, and shaded boxes, respectively. A thick filled bar shows probe A, used in Southern blot analysis for screening genomic DNA. Arrows mark the position and direction of synthesis of oligonucleotides used in PCR genotyping. B, *Bam*HI*;* P, *Pst*I*; H, Hind*III*;* E, *Eco*RI*;* S, *Sma*I*;* X, *Xba*I*;* N, *Not*I*; K, Kpn*I*;* O, *Xho*I*.*

Figure 1 (b) presents a representative Southern blot analysis of BamHI-digested tail DNA from WT (+/+), heterozygous (+/-), and homozygous (-/-) CCR6 mice using probe A. Band sizes in kilobases for the WT and knockout alleles are indicated on the left.

Figure 1(c) presents a representative Northern blot analysis of the CCR6 mRNA expression in thymus and spleen from CCR6 WT (+/+) and knockout (-/-) animals. The size of the CCR6 transcript in kilobases is indicated on the left.

Three independent correctly targeted ES were used to produce chimeric mice by aggregation with CD 1 morulae, which were then transferred to pseudopregnant CD 1 females, as described elsewhere [M. Torres, 1998, The use of embryonic stem cells for the genetic manipulation of the mouse, Curr. Top. Dev. Biol. 99 (1998)]. Chimeric males were bred to C57BL/6 females, and offspring genotype was analyzed by Southern blotting and PCR with specific primers. Transcription of the CCR6 gene was analyzed by Northern blotting, using a DNA fragment internal to the CCR6 coding sequence as probe. No phenotypic differences were detected between the two CCR6-/- mouse lines generated. Several organs, including spleen, thymus, and lymph nodes (LNs), and their resident leukocyte populations were examined, and no differences were found between CCR6-/- mice and the corresponding WT animals. The hematological and bone marrow profiles of WT and CCR6-/- mice were also similar.

*Cell preparations.* Mice were sacrificed by cervical dislocation. Inguinal, axillary, and brachial (IAB) lymph nodes (LNs), mesenteric LNs, Peyer's patches, spleen, and small intestine were collected. The organs were gently disrupted in RPMI 1640 medium, (Bio Whittaker, Verviers, Belgium) with 10% fetal calf serum (FCS) and filtered through nylon mesh to remove aggregates. Splenocytes were depleted of erythrocytes by lysis with 0.83% ammonium chloride. To prepare CD4 T cell-enriched samples, splenocytes were depleted of CD8⁺ T cells and B220⁺ cells with Dynabeads (Dynal Biotech, Oslo, Norway), following manufacturer's instructions. CD8⁺ T cell preparations (>98% pure) were obtained by positive selection using specific Dynabeads. For adoptive transfer experiments in the delayed-type hypersensitivity assays, CD4+ T cell-enriched preparations were injected into the tail vein of recipient mice (2 x 10⁷ cells/mouse). After 16 hours, mice were challenged by injecting 1.3 x 10⁷ BALB/c splenocytes (without red blood cells) into the right footpads, and swelling was monitored over the following days. CD4⁺CD45RB^{high} and CD4⁺CD45RB^{low} T cells (>98% pure) were isolated from the CD4⁺ T cell-enriched population by positive selection using anti-mouse CD4 and CD45RB antibodies (BD Pharmingen) in a Beckman Coulter Epics Altra sorter (Coulter Electronics, Hialeah, Florida, USA). For cell transfer across an MHC class I or II mismatch, CD4⁺CD45RB^{high} (3.5 x 10⁵) or CD8⁺ (2.5 x 10⁶) T cells from WT or CCR6-deficient C57BL/6J mice were injected via the lateral tail vein to sublethally-irradiated (6 Gy) age- and sex-matched C57BL/6J bm1 or bm12 recipients, 2-4 h after irradiation. Recipients were maintained on acidified antibiotic water for 3 weeks after transplant. For cell transfer across mhc mismatches, CD4+CD45RB^{high} (3 x 10⁵) T cells, alone or with CD4⁺CD45RB^{low} (3 x 10⁵) T cells from WT or CCR6-deficient mice (H-2^{d} haplotype-selected CD1 strain), were injected into the tail vein of CB17-Prkdc^{SCID}/J (CB17/SCID) recipients.

*Flow-cytometry studies.* The following FITC-, phycoerythrin- (PE-), Tri-color-(TC-), or SpectraIRed- (SPRD-) conjugated monoclonal antibodies (mAb's) from PharMingen (San Diego, California, USA) were used in this work: anti-CD4 (H129.19), anti-CD8 (53-6.7), anti-B220 (RA3-6B2), and anti-CD45 (30F11). Cell staining and flow cytometry were performed in an EPICS XL flow cytometer (Coulter Electronics Ltd., Hialeah, Florida, USA), according to standard protocols.

*Skin explants.* Ear skin was split into dorsal and ventral portions, and epidermal and dermal sheets were prepared from dorsal portions, before (fresh skin) or after 72-hour culture in complete RPMI-1640 media (skin explants), as described (C. P. Larsen et al., 1990, Migration and maturation of Langerhans cells in skin transplants and explants. J Exp. Med.172:1483-1493).

*Contact hypersensitivity.* Mice were sensitized topically by applying 25 microliters of 0.5% 2,4-dinitrofluorobenzene (DNFB; Sigma Chemical Co.) solution in acetone/ olive oil (4: 1) to the shaved abdomen. Five days later, 20 microliters of 0.2% DNFB in the same vehicle was applied to the right ears, and vehicle alone to the left ears. Ear thickness was measured with a dial thickness gauge (Mitutoyo Corp., Kawasaki, Japan), and ear swelling was estimated by subtracting the pre-challenge from the post-challenge value, and by further subtracting any swelling detected in the vehicle-challenged contralateral ear.

*Delayed-type hypersensitivity.* Mice were sensitized by intravenous injection of 10⁶ BALB/c splenocytes, and challenged on day 5 with 13 x 10⁶ BALB/c splenocytes (50 µl PBS) in the right footpads. Control left footpads received 50 µl PBS. Right footpad swelling was calculated on different days by subtracting the pre-challenge value and any swelling measured in left footpads from the post-challenge value.

*In vitro antigen-presentation assays.* In vitro lymphocyte proliferation in response to 2,4-dinitrobenzenesulphonic acid (DNBS), the water-soluble analogue of dinitrofluorobenzene (DNFB), was determined. Five days prior to cell collection, CCR6-/- knockout and WT littermate control mice were sensitized topically by applying 25 microliters of a 0.5% DNFB solution to the shaved abdomen. Vehicle alone was applied to untreated mice. IAB and mesenteric LNs from sensitized or untreated mice were collected and pooled. LN cells were then cultured in the presence or absence of DNBS (50 µg/ml). Polyclonal activation of lymphocytes was performed by incubating LN cells with 5 µg/ml of Con A (Sigma Chemical Co.) for 48 hours. All cultures were then pulsed with 37000 Bq/well of ³H-thymidine for 18 hours.

Allogeneic mixed lymphocyte reactions (MLR) were performed as follows: Stimulating cells were BALB/c splenocytes, inactivated with 50 µg/ml of mitomycin C (37°C, 20 minutes). Responding cells were WT or CCR6-/- lymphocyte pools from IAB LNs. Stimulating and responding cells were co-cultured (37°C, 72 hours) at different ratios, then pulsed with 37000 Bq/well of ³H-thymidine for 24 hours.

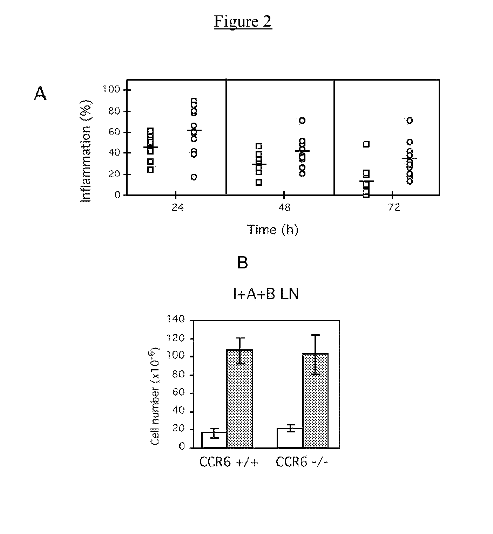

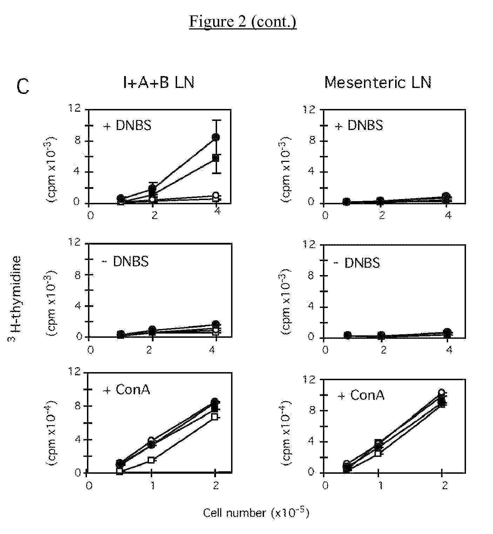

*DNFB-induced contact hypersensitivity.* Figure 2(a). CCR6-/- (n = 12; circles) and CCR6+/+ (n = 12; squares) mice were sensitized by epicutaneous application of 25 µl of 0.5% DNFB solution on the shaved abdomen. Increases in ear swelling were measured 24, 48, and 72 hours after challenge with 20 µl of 0.2% DNFB on the ears. Individual data and the mean value for each group are presented. Two-tailed t-test values for DNFB data: P = 0.006 (24 hours), P = 0.005 (48 hours), P = 0.002 (72 hours).

Figure 2(b) shows that DNFB-induced contact hypersensitivity inflammation is not a function of differences in cell number. Similar number of lymph node cells were present in both CCR6+/+ and CCR6-/- mice. Animals were sensitized with DNFB (filled bars) or left untreated (open bars); 5 days later, lymphocytes from inguinal, axial and brachial (I+A+B) LNs were prepared and counted.

Figure 2(c) shows CCR6-/-lymphocyte proliferation in response to specific and nonspecific stimuli. CCR6-/- and CCR6+/+ animals were sensitized with DNFB (filled symbols) or untreated (open symbols). Five days post sensitization, cells from draining inguinal, axial and brachial (I+A+B) or control mesenteric LNs were recovered and cultured for 36 hours alone or in the presence of DNBS, as indicated. Cultures were pulsed with ³H-thymidine for 18 hours, and cell proliferation was estimated. Similar experiments were performed to measure in vitro responses to a polyclonal stimulus, Con A. Each value represents the average of three separate groups, consisting of pooled LNs from two mice. All assays were performed in triplicate.

The present invention provides a method to assay for a test compound that moderates CCR6-dependent, DNFB-type contact hypersensitivity inflammation. The method uses as a control a CCR6 knockout animal as provided in the present invention. A preferred knockout animal is a mouse. A preferred knockout mouse is a C57BL/6 mouse having deletions in both CCR6 alleles. The method uses an animal possessing an intact, functional CCR6 allele. A preferred control animal possesses two intact, functional CCR6 alleles. A preferred control animal is a C57BL/6J WT mouse.

Mice are sensitized by applying a solution of DNFB to an area of skin. A preferred area of skin is the abdomen. Preferably, the abdomen is shaved prior to application of DNFB. The solution may comprise a vehicle that dissolves DNFB. A preferred vehicle comprises a mixture of acetone and olive oil. A more preferred vehicle is a 4:1 (by volume) ratio of acetone/olive oil. The solution may comprise up to 1% (v/v) DNFB. A preferred solution comprises 0.05% DNFB.

Five days post sensitization, mice are challenged by applying a test volume of a solution of DNFB to an ear. Preferably, the solution comprises a vehicle of substantially the same composition as the vehicle of the sensitization solution. The solution may comprise up to 0.5% DNFB. Preferably, the solution comprises 0.2% DNFB. Preferably, a volume of vehicle is applied to the contralateral ear. Preferably, a volume of vehicle, substantially identical to the test volume is applied to the contralateral ear.

The method further comprises administering a test compound to the WT mice. The ligand may be administered before, after, or simultaneously with the challenge.

A test compound may be administered systemically. A preferred route of systemic administration is orally. A more preferred route of systemic administration is by injection. A test compound may be administered topically. By way of non-limiting example, a test compound may be dissolved in a vehicle and dropped or swabbed on an exposed area of skin.

The method further comprises comparing a contact hypersensitivity inflammation response of a WT mouse in the presence or absence of said test compound. A preferred response is ear swelling.

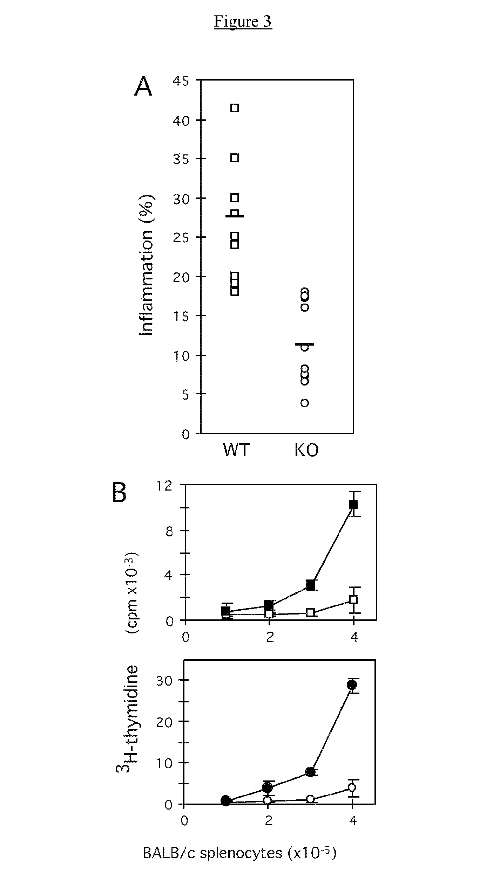

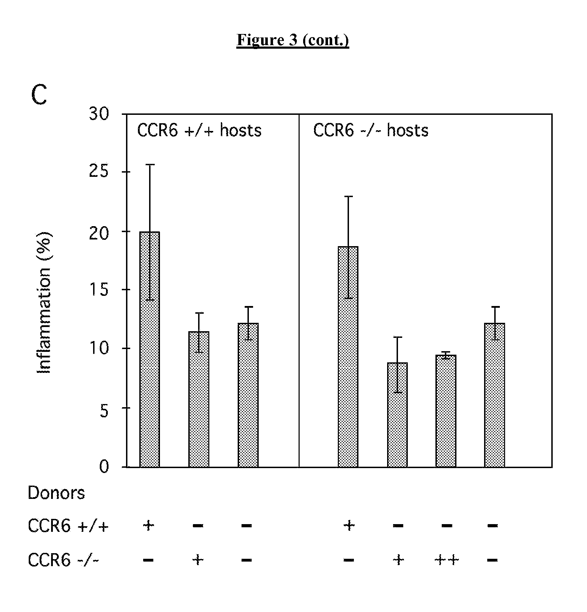

*CCR6-l mice have a markedly diminished DTH response to allogeneic BALB*/*c splenocytes.* Figure 3(a) shows a delayed-type hypersensitivity (DTH) response of CCR6 knockout and WT mice. CCR6-/- (n = 10; circles) and CCR6+/+ (n = 11; squares) animals were sensitized by intravenous injection of 10⁶ BALB/c splenocytes. Five days later, mice were challenged by injecting 13 x 10⁶ BALB/c splenocytes into their right footpads, and swelling was measured 24 hours later. Individual data and the mean value for each group are presented. The two-tailed t-test value for these data was P = 0.000016. Figure 3(b) shows CCR6 knockout and WT, *in vitro* MLR to allogeneic BALB/c splenocytes.

*In vitro MLR to allogeneic BALB*/*c splenocytes.* Lymphocytes were prepared from IAB LNs from CCR6+/+ (filled squares) and CCR6-/- mice (filled circles) and cultured in 96-well plates (2 x 10⁵ cells/well) with increasing amounts of stimulator allogeneic BALB/c splenocytes. After 72 hours, cultures were pulsed with ³H-thymidine for 24 hours and cell proliferation was estimated. Background proliferation is also shown (open symbols). Each point represents the average value of two separate groups, each consisting of pooled LNs from two mice. Assays were performed in triplicate.

Figure 3(c) shows that adoptive transfer of sensitized CCR6+/+ CD4+ T cells to CCR6-/- mice restores their ability to produce a DTH response. Unsensitized CCR6+/+ and CCR6-/- mice were adoptively transferred with 2 x 10⁷ CD4+ T cell-enriched preparations purified from sensitized CCR6+/+ and CCR6-/- donors, as indicated. T cells from CCR6-/- IAB LNs were also allowed to proliferate in an in vitro MLR assay with BALB/ c splenocytes before being injected to CCR6-/- hosts (++). After 16 hours, animals were challenged with 13 x 10⁶ BALB/c splenocytes in footpads, and swelling was measured 24 hours later.

The present invention provides a method to assay for a test compound that moderates CCR6-dependent, DTH inflammation. The method uses as a control a CCR6 knockout animal as provided in the present invention. A preferred knockout animal is a mouse. A preferred knockout mouse is a C57BL/6 mouse having deletions in both CCR6 alleles. The method uses an animal possessing an intact, functional CCR6 allele. A preferred control animal possesses two intact, functional CCR6 alleles. A preferred control animal is a C57BL/6J WT mouse.

The present invention provides a method to assay for a test compound that moderates a CCR6-dependent delayed-type hypersensitivity inflammation response. An aspect of the method provides CCR6 knockout and WT mice.

A further aspect of the present invention provides that the mice are sensitized by intravenous injection of allogeneic splenocytes. Preferred, but non-limiting, splenocytes are obtained from BALB/c mice.

In yet a further aspect, the mice are challenged by injection of allogeneic splenocytes into a footpad. It is preferable that the challenge injection be administered about five days post sensitization.

In an aspect, a footpad swelling in WT mice is compared in the presence and absence of a test compound.

The test compound may be administered systemically as, for example, by injection. The test compound may be administered before, after, or simultaneously with sensitization.

*CCR6 in inflammatory bowel disease (IBD).* Mice with a targeted deletion of the CCR6 gene were generated as described and backcrossed onto a C57BL/6J background for eight generations. C57BL/6J WT littermates were used as controls. BALB/c mice were purchased from the Jackson Laboratory (Bar Harbor, Maine, USA).

*Dextran sodium sulfate (DSS)-mediated colitis.* Colitis was induced by addition of 2.5% (w/v) DSS (M.W. 40,000; ICN Biomedicals, Aurora, Ohio, USA) to drinking water for 7 days [H. Mashimo, D. C. Wu, D. K. Podolsky, and M. C. Fishman, Impaired defense of intestinal mucosa in mice lacking intestinal trefoil factor, Science 1996. 274: 262-265). DSS administration was then stopped and mice received water alone for 3 weeks, after which 2.5% DSS was re-introduced to drinking water for another 3 days. Dosage was established empirically to induce moderate to severe colitis, minimizing mortality. Mean DSS/water consumption, body weight and stool consistency were recorded every 2 days. Fecal blood was assessed using the Hematest System (Bayer Corp., Pittsburgh, PA).

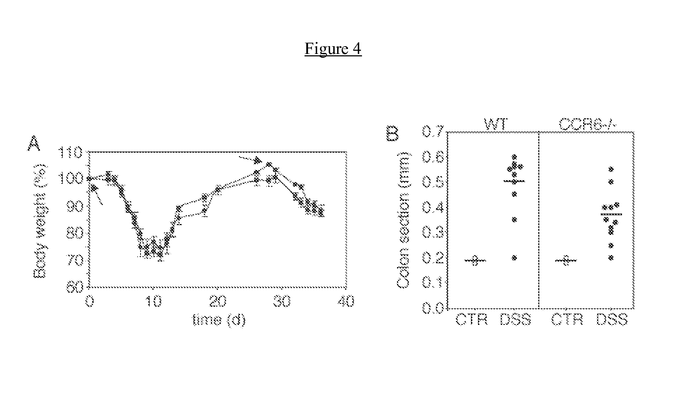

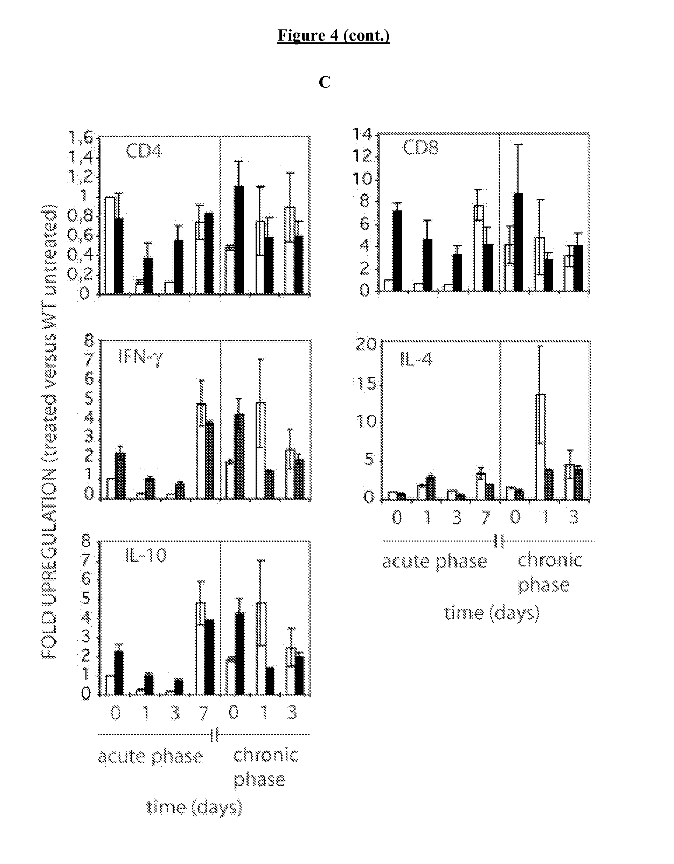

Figure 4(a) shows that CCR6 deficiency protects against DSS-mediated colitis weight loss. Mouse weight was monitored every 2 days. WT (n=18; circles) and CCR6-deficient mice (CCR6^{-/-}; n=18; squares) were treated with 2.5% (w/v) DSS for 7 days (acute phase), followed by a 3-week rest period and re-administration of DSS (chronic phase). Arrows indicate days 0 and 28, when DSS was added to drinking water.

Figure 4(b) shows that CCR6 deficiency protects against DSS-mediated colitis intestinal inflammation. Distance was measured from the muscular propria to the intestinal lumen as an estimation of intestinal inflammation at day 7 in control (CTR) and DSS-treated (DSS) WT and CCR6^{-/-} mice. Individual data and the mean (bar) for each group are shown (p=0.00377; two-tailed t-test).

Figure 4(c) shows altered cytokine response to DSS treatment in CCR6-deficient mice. Colon samples from WT (n=6; gray) and CCR6^{-/-} (n=5; black) mice were obtained on the days indicated during acute and chronic phases of DSS-induced colitis, and processed for real-time quantitative RT-PCR analysis of CD4, CD8 and cytokine transcripts. Representative results are shown from two experiments.

To measure cytokine production in response to DSS administration, whole colon tissue samples were analyzed by real-time quantitative RT-PCR. At day 0, basal levels of IFN-γ and IL-10 were about twofold higher in CCR6-deficient than in WT mice (Fig. 4c). During the acute colitis phase (days 1-7), there was no marked variation in IFN-γ mRNA production in WT or CCR6-deficient animals, although WT mice showed an increase in IFN-γ transcripts (Fig. 4c). In the chronic disease phase there was nonetheless a clear Th1 response in WT mice, as deduced from the notable increase in IFN-γ mRNA levels (three- to fourfold) just 24 h after DSS re-administration (Fig. 4c). In contrast, DSS re-administration to CCR6-deficient mice provoked no significant changes in IFN-γ transcript levels, which did not exceed the basal levels observed in untreated CCR6-deficient animals (Fig. 4c). These data suggest that there is no specific Th1 cytokine response to DSS administration in CCR6-deficient animals.

We also measured IL-4 and IL-10 transcript levels in colon samples to estimate the cytokine response following DSS treatment. Basal IL-10 mRNA levels were higher in CCR6-deficient mice (about twofold), decreasing at days 1 and 3 of DSS treatment in both animal groups, particularly in the WT animals (Fig. 4c); this may be due to destruction of immune cells resident in the mucosal tissue subsequent to DSS administration. In any case, absolute IL-10 transcript levels remained higher in CCR6-deficient than in WT mice on days 1 and 3, recovering to a similar level in both mouse groups by day 7 (Fig. 4c). In the chronic phase, WT mice responded with an immediate increase in IL-10 mRNA levels (about fourfold, day 1; Fig. 4c). The response differed in CCR6-deficient mice, as the IL-10 transcript level decreased slightly as in the acute phase (Fig. 4c), suggesting that acute-phase immunization of these animals was less efficient than in WT mice. IL-4 mRNA up-regulation in the chronic phase was stronger and more rapid in the WT mouse group than that observed in CCR6-deficient mice (day 1; Fig. 4c).

*TNBS-induced colitis.* Mice received enemas of TNBS (Sigma, St. Louis, Missouri, USA) dissolved in 50% ethanol in phosphate buffered saline (PBS), given under isofluorane anesthesia using a tuberculin syringe fitted with a 2.5-cm gastric intubation needle. Control mice received enemas of 50% ethanol in PBS. Mice were sensitized three times at weekly intervals (2 mg for initial treatment, 1 mg for subsequent doses) [M. F. Neurath, I. Fuss, B. L. Kelsall, E. Stuber and W. Strober, Antibodies to interleukin 12 abrogate established experimental colitis in mice, J. Exp. Med. 1995, 182: 1281-1290]. At 5-7 days after the third TNBS administration, mice were killed and analyzed as described below.

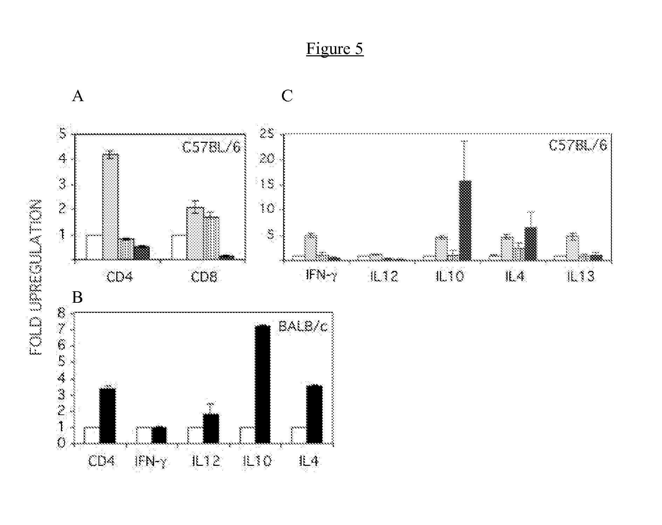

Figure 5 shows that TNBS treatment provokes distinct T cell and cytokine responses in WT and CCR6-deficient C57B/6J mice. Seven days after administration of the last enema, samples of distal half colons were obtained from control (PBS; n = 6) or TNBS-treated mice of the indicated genetic backgrounds and genotypes: BALB/c, n = 6 (PBS, white; TNBS, black); C57BL/6J WT, n=8 (PBS, white; TNBS, striped); C57BL/6J CCR6^{-/-}, n = 7 (PBS, gray; TNBS, black). Samples were processed for real-time quantitative RT-PCR analysis of CD4 and CD8 (a) and cytokine transcripts (b, c) as indicated. Representative results are shown from two experiments.

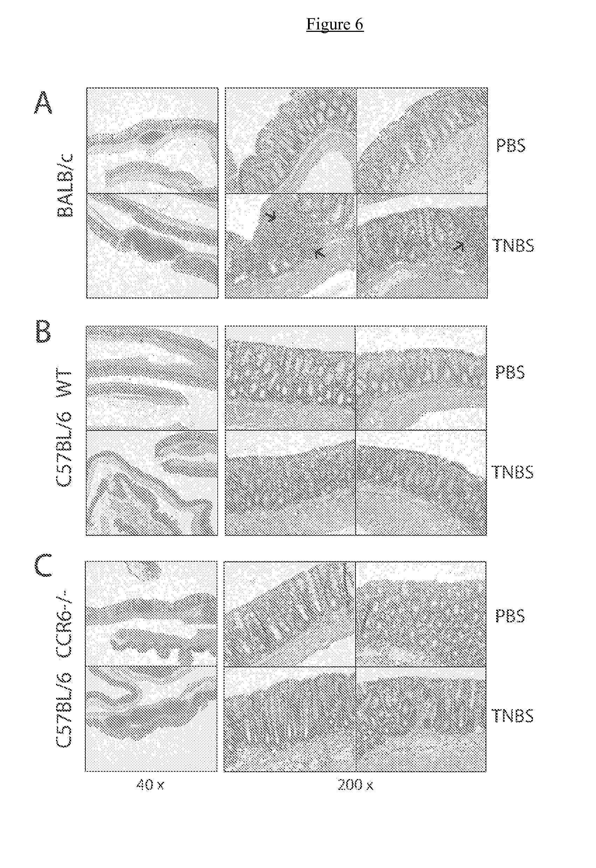

Figure 6 shows that CCR6-deficiency confers susceptibility to TNBS-induced colitis in the normally resistant C57BL/6J mouse strain. Colon tissue sections from control (PBS) or TNBS-treated (TNBS) animals were obtained at day 7 after the last enema and stained with hematoxylin/eosin. Photomicrographs (left column, 40x; middle and right column, 200x magnification) show different aspects of the TNBS-mediated colitis and are representative of two experiments performed. Locally extensive inflammatory reaction forming microgranulomas in TNBS-treated BALB/c mice are marked by arrows.

*Colitis assessment.* The general procedure was as described elsewhere (P. G, Andres, P. L. Beck, E. Mizoguchi, A. Mizoguchi, A. K. Bhan, T. Dawson, W. A. Kuziel, N. Maeda, R. R. MacDennott, D. K. Podolsky and H. C. Reinecker, Mice with a selective deletion of the CC chemokine receptors 5 or 2 are protected from dextran sodium sulfate-mediated colitis: lack of CC chemokine receptor 5 expression results in a NK1.1+ lymphocyte-associated Th2-type immune response in the intestine, J. Immunol. 2000, 164: 6303-6312). Animals were sacrificed by CO₂ narcosis and the entire colon was removed. Colon length and number of adhesions between colon and other peritoneal structures were recorded. Colitis extent and severity were assessed macroscopically by clinical scoring of the percentage of the colon and cecum showing macroscopic evidence of colitis, as follows: 0, normal; 1, erythema; 2, presence of ulcers; and 3, diffuse ulceration. Tissue sections from the entire colon were examined and scored in a blind fashion for severity and extent of ulceration. Tissue thickness was determined from the muscularis propria to the luminal border. Lesion severity was graded according to a previously defined scoring system: 0, normal; 1, mild; 2, moderate; and 3, severe. (P. G, Andres, P. L. Beck, E. Mizoguchi, A. Mizoguchi, A. K. Bhan, T. Dawson, W. A. Kuziel, N. Maeda, R. R. MacDennott, D. K. Podolsky and H. C. Reinecker, Mice with a selective deletion of the CC chemokine receptors 5 or 2 are protected from dextran sodium sulfate-mediated colitis: lack of CC chemokine receptor 5 expression results in a NK1.1+ lymphocyte-associated Th2-type immune response in the intestine, J. Immunol. 2000, 164: 6303-6312). Mild lesions contained small, focal, or widely dispersed areas of inflammation and/or fibrosis above the muscularis mucosa. Moderate lesions were multifocal or locally extensive and showed inflammation or fibrosis extending into the submucosa. Severe lesions had inflammatory cells extending into the muscularis propria. Colitis onset and progress were monitored by registering rectal bleeding, diarrhea and body weight loss every 2 days.

*CCR6 deficiency confers susceptibility to TNBS-induced colitis in normally resistant C57BL*/*6J mice.* Administration of TNBS per rectum in the presence of ethanol induces a mucosal immune response leading to large intestine inflammation in susceptible (BALB/c) but not in resistant (C57BL/6J) mouse strains ( C. O. Elson, K. W. Beagley, A. T. Shannanov, K. Fujihashi, H. Kiyono, G. S. Tennyson, Y. Cong, C. A. Black, B. W. Ridwan and J. R. McGhee, Hapten-induced model of murine inflammatory bowel disease: mucosa immune responses and protection by tolerance. J. Immunol. 1996, 157: 2174-2185]. The strain used also influences disease manifestations; TNBS-mediated colitis thus has a Th1 character in the SJL/J mouse strain, whereas it shows a Th2 (or mixed Th1/Th2) character in the BALB/ c background (W. Strober, I. J. Fuss, R. S. and Blumberg, The immunology of mucosal models of inflammation, Annu. Rev. Immunol. 2002, 20: 495-549).

We studied whether the CCR6 deficiency-provoked alterations in lymphocyte homeostasis and basal Th1/Th2 cytokine levels in intestinal mucosa were sufficient to modify C57BL/6J resistance to TNBS-induced colitis. To emulate a chronic disease state, animals received three enemas of TNBS in 50% ethanol at weekly intervals. Compared to control animals, which received PBS/ethanol enemas and showed histologically normal colons, susceptible BALB/c mice developed diffuse lymphocytic colitis of moderate intensity (grade 2; Figure 6a) and severe hypertrophy of gut-associated lymphoid tissue (Figure 6a). This was accompanied by mucosal inflammation that affected mainly the distal half of the colon, characterized by a multifocal or locally extensive inflammatory reaction composed of leukocytes and macrophages that formed microgranulomas (Figure 6a, arrows) that distended the lamina propria and separated the crypts. Some increase in lymphocyte number was also observed in submucosa. No edema or ulceration was present (Figure 6a).

On the C57BL/6J background, clear differences were found between TNBS-treated WT and CCR6-deficient mice. WT C57BL/6J mice behaved as a resistant strain, with mild hypertrophy of gut-associated lymphoid tissue and almost no inflammatory infiltrate in epithelia and lamina propria (Figure 6b). Conversely, CCR6-deficient C57BL/6J mice had mild diffuse lymphocytic colitis, with increased numbers of immune cells infiltrating the lamina propria and submucosa (Figure 6c). In addition, hypertrophy of gut-associated lymphoid tissue was seen in colonic sections (Figure 6c). Although the inflammatory process was milder in CCR6-deficient C57BL/6J than in BALB/c mice, the disease in CCR6-deficient mice was more wasting than in WT C57BL/6J mice, which were practically resistant.

As TNBS colitis is a T cell-mediated process (W. Strober, I. J. Fuss, and R. Blumberg, The immunology of mucosal models of inflammation. Annu. Rev. Immunol. 2002, 20: 495-549), we used a real-time quantitative RT-PCR procedure to compare CD4 and CD8 mRNA expression in mouse colons 7 days after administration of the last enema. No major changes were observed in TNBS-treated WT C57BL/6J mice, except for a mild increase in CD8 transcript levels (Figure 5a). In contrast, CCR6-deficient C57BL/6J mice had higher basal levels of both CD4 (about fourfold) and CD8 (about twofold) transcripts after control PBS/ethanol enema treatment; these levels decreased by about tenfold after hapten treatment. In BALB/c mice, TNBS treatment induced an about threefold increase in CD4 mRNA (Figure 5b), which may correspond to CD4 T cell infiltration *or in situ* proliferation.

*TNBS administration-induced cytokine response in the colon.* To analyze the T cell response in TNBS-mediated colitis, we measured cytokine mRNA levels in the distal half of the colon 7 days after administration of the last enema. Real-time quantitative RT-PCR assays showed that the cytokine profile of CCR6-deficient C57BL/6J mice was equivalent to that of BALB/c mice and differed from that of WT C57BL/6J mice (Figures 5b, c). Consistent with the inflammation developed and the mixed Th1/Th2 character described in TNBS colitis, IL-12, IL-10, and IL-4 transcript levels were higher in colons of TNBS-treated than in control-treated BALB/c mice. In CCR6-deficient C57BL/6J mice, baseline mRNA levels of IFN-α, IL-10, IL-4, and IL-13, but not IL-12, were about fourfold higher than those of WT C57BL/6J animals, which are resistant to TNBS-induced colitis. In addition, increased levels of IL-10 and IL-4 cytokine transcripts were observed in CCR6-deficient C57BL/6J mice after TNBS treatment (Figure 5c).

Graft-versus-host disease (GvHD) is a common, debilitating complication of allogeneic bone marrow transplantation [V. T. Ho and R. J. Soiffer, The history and future of T-cell depletion as graft-versus-host disease prophylaxis for allogeneic hematopoietic stem cell transplantation, Blood 98: 3192-3204 (2001)]. The disease is a multistep process involving lymphocyte activation, proliferation and target cell killing. Donor T cells recognize the major histocompatibility complex (MHC) and associated peptides on host-derived antigen-presenting cells. This leads to cell activation and differentiation into various effector and memory T cells with selective tropism [F. Sallusto, D. Lenig, R. Forster, M. Lipp and A. Lanzavecchia, Two subsets of memory T lymphocytes with distinct homing potentials and effector functions. Nature 401,708-712 (1999); J. H. Antin, Acute graft-versus-host disease: inflammation run amok? J. Clin. Invest. 107, 1497-1498 (2001)]. In acute GvHD, activated T cells infiltrate mainly the skin, liver, and gastrointestinal tract, where they initiate a Th1-mediated immune response [D. Snider and H. Liang, Early intestinal Th1 inflammation and mucosal T cell recruitment during acute graft-versus-host reaction, J. Immunol. 166, 5991-5999 (2001)]. IFN-γ collaborates in recruitment of other T cell subsets, monocytes and macrophages that further damage affected tissues (W. Krenger and J. L. Ferrara, Graft-versus-host disease and the Th1/Th2 paradigm, Immunol. Res. 15, 50-73 (1996); W. D. Shlomchik et al., Prevention of graft versus host disease by inactivation of host antigen-presenting cells, Science 285, 412-415 (1999)]. Recent data showing that neutralization of TNF-α and IL-1 prevents acute GvHD confirm the central role of inflammatory cytokines in this disease [T. Teshima et al., Acute graft-versus-host disease does not require alloantigen expression on host epithelium, Nat. Med. 8: 575-581 (2002)].

We analyzed the role of CCR6 in acute GvHD development in MHC-mismatched recipients. Results show that disease onset was delayed and the mortality ratio decreased in MHC class II-mismatched recipients of CD4+ T cells from CCR6-deficient compared to WT donors. Conversely, CD8+ T cells from both CCR6-deficient and WT donors provoked similar GvHD symptoms in MHC class 1-mismatched recipients. CCR6 was also critical for GvHD development across minor histocompatibility complex (mhc)-mismatched barriers. Onset of skin lesions and intestinal inflammation was delayed and symptoms were milder in MHC-matched/mhc-mismatched recipients of naive CD4⁺CD45RB^{high} T cells from CCR6-deficient donors. Significant delay was observed in CD45⁺ and CD4⁺ cell infiltration to skin and intestine in recipients of naive T cells from CCR6-deficient donors. Lower IFN-α and IL-1 0 levels were found in skin and gut of these mice, and levels of the chemokines that control activated T cell homing to skin and gut were also decreased. Our data suggest that by facilitating alloreactive effector/memory CD4⁺ T cell recruitment to target tissues, CCR6 plays a major role in GvHD development.

*Lack of CCR6 activity in donor T cells protects MHC class II-mismatched recipient mice from GvHD.* To study the role of CCR6 in GvHD development, we transferred T cells from C57BL/6J WT or CCR6-deficient donors to sub-lethally irradiated (6 Gy) bm1 and bm12 mice, which differed from donor mice at a single MHC class I (BALB/C B6.C-H2^{bm1}/By; bm1) or class II (BALB/c B6.C-H2-2^{bm12}/KhEg; bm12) allele. Injection of WT CD4⁺CD45RB^{high} (naive) lymphocytes into bm12 mice produced acute GvHD. Animals began dying shortly after cell transplant (ACT) and all mice had died by day 18 (Figure 7a).

When naive T cells from CCR6-deficient donors were transferred, however, we observed a significant decrease in mortality of recipient bm12 mice; 40% of transplanted animals were alive 70 days ACT (Figure 7a). Mortality onset was also delayed in this group, as mice did not begin to die until day 17 ACT (Figure 7a). Weight loss at day 15 ACT was significantly lower in bm12 mice that received T cells from CCR6-deficient donors compared to recipients of WT cells (Figure 7d).

Figure 7 shows that the transfer of CD4⁺ T cells from CCR6-deficient mice to MHC class II-mismatched recipients results in milder GvHD. Survival (a, b, c) and animal weight at indicated days (d, e, f) of bm1 and bm12 (n = 8) mice recipients of WT (o) and CCR6-deficient (□) CD4⁺ and CD8⁺ T cells, as indicated. Recipient weight is also shown prior to cell transfer (Δ).

An aspect of the present invention provides a method to assay for a test compound that moderates CCR6-dependent GvHD comprising providing CD4⁺ T cells from a CCR6 knockout mouse as a control; providing CD4⁺ T cells from a CCR6 WT mouse; providing a CCR6 WT, MHC(II)-mismatched mouse; providing a test compound; transferring said T cells to said WT mouse in the presence and absence of said test compound; and comparing a GvHD response in the presence and absence of said test compound.

A further aspect of the present invention provides convenient, non-limiting GvHD responses include percent survival at a pre-determined time post transfer (Figure 7a), recipient weight at a pre-determined time post transfer (Figure 7d), ear inflammation (Figure 8a), skin lesions (Figure 8b), diarrhea (Figure 8d), spleen index, and CD4⁺ T cell expansion.

Figure 7a shows that percent survival may be described by a sigmoid curve as a function of time post transfer. The time post transfer at which percent survival is determined may be any day post transfer. A preferred day is day fifteen post transfer. A more preferred time to determine percent survival is at an inflection point on a survival curve. The inflection point may relate to the curve describing survival of WT or CCR6-deficient animals.

In contrast, 100% of bm1 mice transplanted with CD8⁺ T cells from WT or CCR6-deficient donors died from acute GvHD 15-16 days ACT (Figure 7b). Similar weight loss was observed in both bm1 mouse groups by day 13 ACT (Figure 7e). Following control cell transfer of CD4⁺ naive T cells from WT or CCR6-deficient donors to bm1 mice, no alterations were detected in body weight or survival for 70 days ACT (Figures 7c, 7f). The results show that WT and CCR6-deficient CD8⁺ T cells have similar ability to cause GvHD across MHC class I barriers.

*GvHD onset is delayed and lesion severity decreased in mhc-mismatched recipients of naive T cells from CCR6-deficient donors.* We analyzed the role of CCR6 in GvHD mediated by CD4⁺ T cells from H-2^{d} haplotype-selected WT or CCR6-deficient mice transferred to MHC-matched/mhc-mismatched CB 17/SCID recipient mice. CD4⁺CD45RB^{high} T cell recipients developed a wasting syndrome associated with splenomegaly, erythrosquamous skin lesions and intestinal inflammation. Ear thickness in recipient mice was evaluated at several time points as an overall measure of skin inflammation, and skin lesions were scored clinically. Weight loss and diarrhea were monitored as an indication of intestinal damage.

As early as 19 days ACT, ear inflammation increased significantly in WT naive T cell recipients (0.370 ± 0.022 mm), a 46% increment over untransplanted control mice (0.261 ± 0.006 mm) (Figure 8a). Subsequent damage to ear tissue in WT naive T cell recipients prevented registering further increases in ear inflammation. By 19 days ACT, however, only a slight increase in ear thickness was observed in T cell recipients from CCR6-deficient donors (0.305 ± 0.024 mm), 17% higher than that of controls (Figure 8a). This inflammation increased with time, peaking at days 30-35 ACT (0.406 ± 0.051 mm, 55% over control).

Skin lesion development differed between groups of cell-transplanted mice. Lesions were first detected on ears and head, later extending to trunk and feet; progression was rapid and homogeneous in WT T cell recipients (Figure 8b). Lesion onset on ears and head was apparent 3 weeks ACT (day 23-24); at 5 weeks, all mice showed the maximum clinical score, with ears, head, trunk and feet affected. Pustules were also observed on the trunk at later stages. By 3 weeks ACT, T cell recipients from CCR6-deficient donors had only developed mild ear inflammation. Lesions in ears and head were not observed until 6 weeks ACT. Disease was progressive thereafter, and 80% of the population reached the maximum score by 9 weeks ACT (day 62).

Adoptively transferred CD4⁺CD45RB^{low} memory/regulatory T cells do not provoke GvHD in recipient mice and are able to suppress GvHD caused by CD4⁺CD45RB^{high} naïve T cells when both cell types are cotransferred (M.P. Schon, M. Detmar and C. M. Parker, 1997, Murine psoriasis-like disorder induced by naive CD4+ T cells, Nat. Med. 3: 183-188; P. J. Morrissey, K. Charrier, S. Braddy, D. Liggitt and J. D. Watson, 1993, CD4+ T cells that express high levels of CD45RB induce wasting disease when transferred into congenic severe combined immunodeficient mice. Disease development is prevented by cotransfer of purified CD4+ T cells. J. Exp. Med. 178: 237-244). Control cell transfers in which CB17/SCID recipients received memory/regulatory T cells from BALB/cJ donors did not provoke any intestinal or cutaneous pathology nine weeks ACT (Figures 8f, g). Cotransfer of naïve and memory T cells showed that WT memory T cells suppressed ear inflammation, skin lesions, diarrhea and weight loss induced by naïve T cells from CCR6-deficient donors in recipient mice (Figures 8a, b, d, e). This regulatory activity was impaired in memory cells from CCR6-deficient mice, which did not prevent development or reduce severity of the cutaneous and intestinal pathologies induced by WT naïve T cells in recipients cotransferred with both cell types (Fig. 8a, b, d, e).

Figure 8 shows that transfer of naive CD4⁺ T cells from CCR6-deficient mice to MHC-matched/mhc-mismatched recipients delays GvHD onset and reduces severity of symptoms, and that the regulatory activity of CCR6-deficient CD4⁺CD45RB^{low} regulatory T cells is impaired. The time course of symptom development is shown for (a), ear inflammation, statistical analysis using Student's t test comparing WT vs. CCR6-deficient naive T cell recipients yielded P = 1.5 x 10⁻⁶ (day 19 ACT) and P = 0.0013 (day 22 ACT); (b), erythrosquamous skin lesions, P < 0.003 x 10⁻⁶ (days 19-34 ACT); (c), representative hematoxylin/eosin-stained dorsal skin sections of T cell recipients from WT or CCR6-deficient donors taken at days 12 and 28 ACT; (d), percentage of mice showing diarrhea; (e), changes in body weight. Mean values ± SD are shown from one experiment representative of three independent transfer experiments in which recipient mice were reconstituted with 0.35 x 10⁶ MHC-matched/mhc-mismatched WT CD4⁺CD45RB^{high} naive T cells (o, n = 37), CCR6-deficient CD4⁺CD45RB^{high} naive T cells (□, n = 57), WT naive plus CCR6-deficient CD4⁺CD45RB^{low} memory/regulatory T cells (•, n = 19), or WT memory/regulatory plus CCR6-deficient naive T cells (■, n = 16).

Results are also shown for ear inflammation (f) and body weight change (g) from control experiments in which naive (△) or memory/regulatory (▲) BALB/c CD4⁺ T cells were transferred to recipient mice.

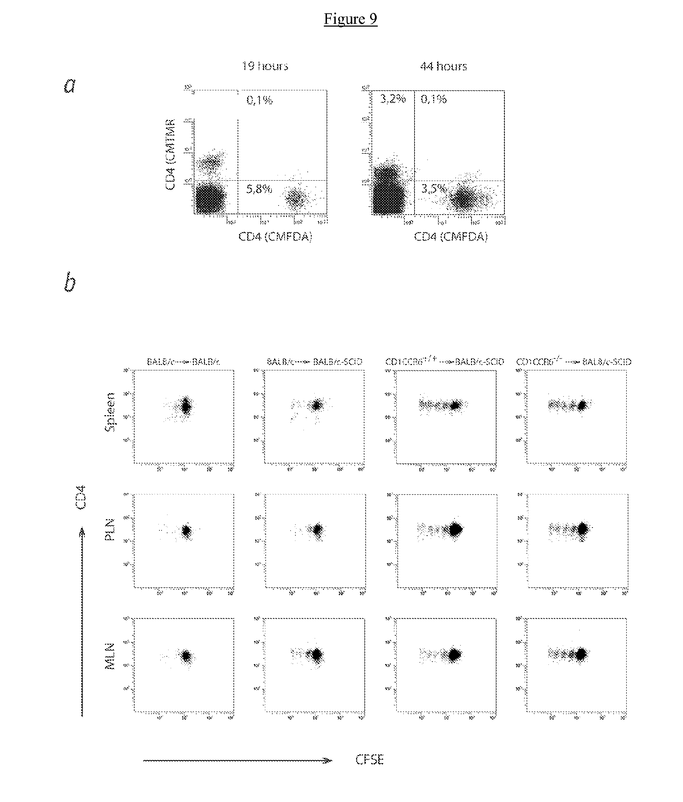

Figure 9 shows that *in vivo* homing of CD4⁺ T cells to secondary lymphoid organs and on-site proliferation is CCR6-independent. CMFDA-labeled WT and CMTMR-labeled CCR6-deficient CD4⁺ T cells (3 x 10⁶ each) were co-transferred into BALB/c-SCID mice and the percentage of donor cells in recipient spleens determined at different times ACT (a). WT or CCR6-deficient CD4⁺ T cells transferred to BALB/c-SCID mice showed a similar proliferation ratio, as deduced from the sequential halving of the fluoresce intensity with each generation of CD4⁺ T cells in spleen, peripheral (PLN) and mesenteric (MLN) lymph nodes. Data are also shown from control transplants in which CFSE-labeled CD4⁺ T cells from BALB/c donors were transferred to BALB/c or BALB/c-SCID recipients. Plots represent cells recovered from 4 mice, 4 days after receiving a transplant of 10 x 10⁶ CFSE-labeled CD4⁺ T cells of the indicated genotypes. Similar results were obtained in three different experiments (b).

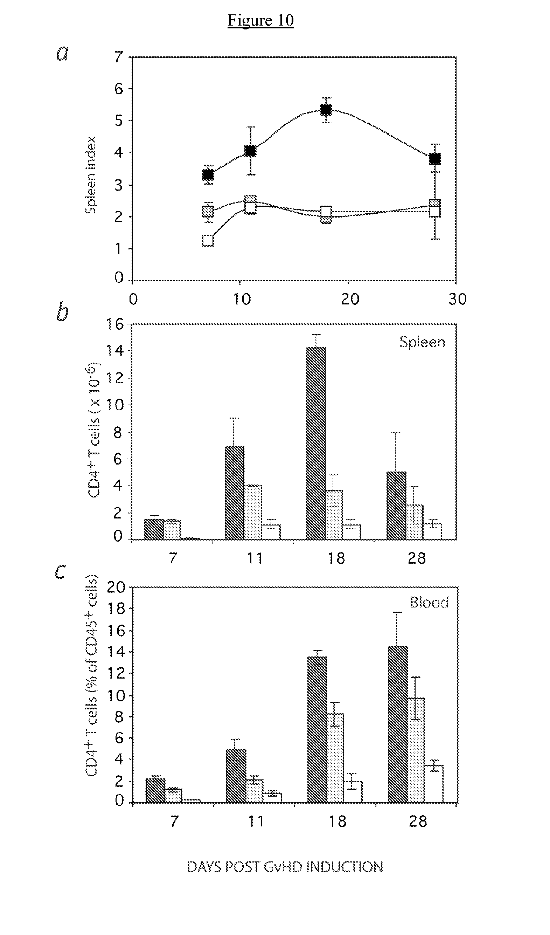

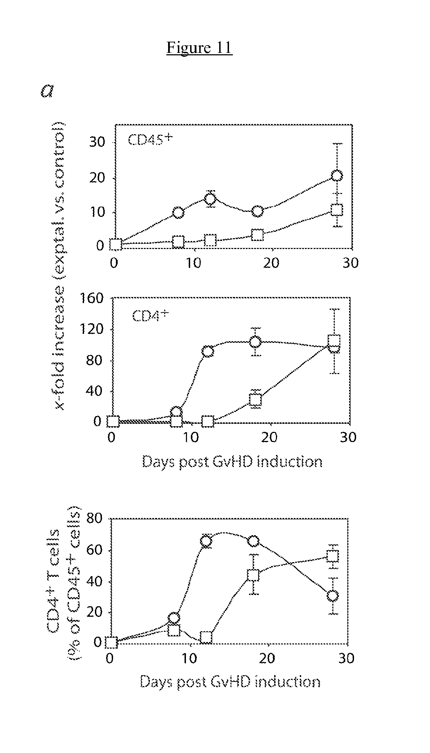

Figure 10 shows that long-term expansion of CD4⁺ T cells is reduced in recipients of CCR6-deficient donor cells: (a) spleen index comparison for the first 28 days after GvHD induction in CB17/SCID recipients of WT (■), CCR6-deficient (■) or BALB/c (□) CD4⁺ naïve T cells. CD4⁺ T cell number is also shown for recipient spleen (b) and blood (c) at different time-points after GvHD induction. Values represent the mean ± SD (n = 2-4 mice/group/time-point). Similar results were observed in two independent experiments.

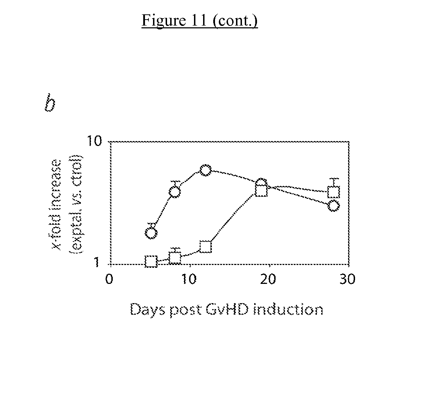

Figure 11 shows delayed CD4⁺ T cell homing to skin and small intestine of recipients of cells from CCR6-deficient donors. (a), Flow cytometric analysis of CD45⁺ and CD4⁺ cells infiltrated in ear skin. (b), Real-time quantitative RT-PCR analysis of CD4 mRNA levels in the small intestine of naive T cell recipients. Data represent the mean ± SD (n = 2-4 mice/group/time-point) and illustrate the x-fold increase in transplanted (o, WT donors; □, CCR6-deficient donors) compared to un-transplanted mice.

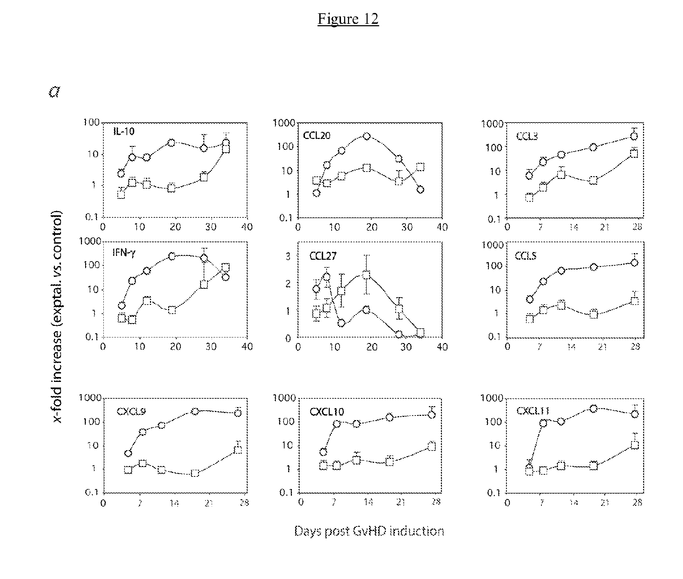

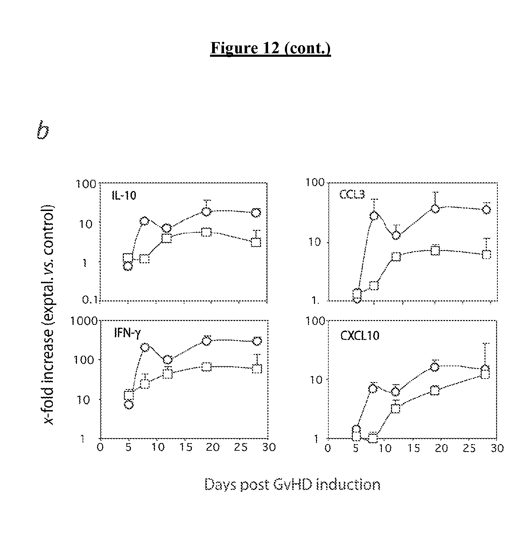

Figure 12 shows that recipients of CD4⁺ naive T cells from CCR6-deficient donors have lower and delayed expression of pro-inftammatory cytokines and chemokines in GvHD target organs. Cytokine and chemokine transcript levels (indicated) were measured over time by real-time quantitative RT-PCR in ear skin (a) and small intestine (b) of naive T cell recipients from WT (o) and CCR6-deficient (□) donors. Data represent the mean ± SD (n = 2-4 mice/group/time-point) and illustrate the x-fold up-regulation observed in transplanted vs. un-transplanted mice.

### INCORPORATION BY REFERENCE

All publications and patent applications cited in this specification are herein incorporated by reference, and for any and all purposes, as if each individual publication or patent application were specifically and individually indicated to be incorporated by reference. In the case of inconsistencies the present disclosure will prevail. Particularly, but not by way of limitation, Varona et al., CCR6-Deficient Mice Have Impaired Leukocyte Homeostasis And Altered Contact Hypersensitivity And Delayed-Type Hypersensitivity Responses, 107 Journal of Clinical Investigation R37 (2001); R. Varona, et al., Molecular cloning, functional characterization and mRNA expression analysis of the murine chemokine receptor CCR6 and its specific ligand MIP-3a. 440 FEBS Lett. 188 (1998); M. Torres, 1998, The use of embryonic stem cells for the genetic manipulation of the mouse, 36 Curr. Top. Dev. Biol. 99 (1998); Varona, et al., CCR6 Has A Non-Redundant Role In The Development Of Inflammatory Bowel Disease, 33 Eur. J. Immunol. 2937 (2003); and Rosa Varona, Vanesa Cadenas, Lucio Gomez, Carlos Martinez-A. and Gabriel Marquez, CCR6 regulates CD4+ T cell-mediated acute graft-versus-host disease responses, submitted for publication; are specifically incorporated by reference in their entirety and for all purposes.

### DEPOSIT OF BIOLOGICAL MATERIALS

Homologous, recombinant embryonic stem cells, CCR6KOES192, containing a CCR6 deletion were deposited on February 11, 2004 under Accession Number DSM ACC2636 at the Deutsche Sammlung von Mikroorganismen und Zellkulturen GmbH, Mascheroder Weg 1b, D-38124 Braunschweig, Germany. Members of the public may access the deposit under conditions set forth in 37 CFR 1.808.

### INDUSTRIAL UTILITY

This invention identifies CCR6 as a target for drugs that reduce inflammatory, immunological responses and has industrial applicability in providing means to assay for test compounds that moderate CCR6-dependent physiological functioning and disease processes based thereon.

### REFERENCES

J. Banchereau et al., 2000, Immunobiology of Dendritic Cells, Annu. Rev. Immunol. 18: 767-811.

R. Forster et al., 1996, A putative chemokine receptor, BLR1, directs B cell migration to defined lymphoid organs and specific anatomic compartments of the spleen, Cell. 87: 1.037-1.047.

F. Sallusto, A. Lanzavecchia y C. R. Mackay, 1998. Chemokines and chemokine receptors in T-cell priming and Th1/Th2-mediated responses, Immunol. Today 19: 568-574.

F. Sallusto, D. Lenig, R. Forster, M. Lipp y A. Lanzavecchia, 1999, Two subsets of memory T lymphocytes with distinct homing potentials and effector functions, Nature, 401: 708-712.

R. Forster et al., 1999. CCR7 coordinates the primary immune response by establishing functional microenvironments in secondary lymphoid organs, Cell, 99: 23-33.

J. G. Cyster, 1999, Chemokines and cell migration in secondary lymphoid organs, Science, 286: 2.098-2.102.

J. J.Campbell y E. C. Butcher, 2000, Chemokines in tissue-specific and microenvironment-specific lymphocyte homing, Curr. Opin. Immunol. 12: 336-341.

M. Baba et al., 1997, Identification of CCR6, the specific receptor for a novel lymphocyte-directed CC chemokine LARC, J. Biol. Chem. 272: 14.893-14.898.

F. Liao et al. 1997, STRL22 is a receptor for the CC chemokine MIP-3α, Biochem. Biophys. Res. Commun. 236: 212-217.

C. A. Power et al., 1997. Cloning and characterization of a specific receptor for the novel CC chemokine MIP-3V from lung dendritic cells, J. Exp. Med. 186: 825-835.

D. R. Greaves et al., 1997, CCR6, a CC chemokine receptor that interacts with macrophage inflammatory protein 3α and is highly expressed in human dendritic cells, J. Exp. Med. 186: 837-844.

D. L. Rossi, A. P. Vicari, B. K. Franz, T. K. McClanahan y A. Zlotnik, 1997, Identification through bioinformatics of two new macrophage proinflammatory human chemokines: MIP-3α and MIP-3β, J. Immunol. 158: 1.033-1.036.

R. Hromas et al., 1997, Cloning and characterization of exodus, a novel β-chemokine, Blood 89: 3.315-3.322.

M. C. Dieu et al., 1998, Selective recruitment of immature and mature dendritic cells by distinct chemokines expressed in different anatomic sites, J. Exp. Med. 188: 373-386.

L. Carramolino et al., 1999, Down-regulation of the β-chemokine receptor CCR6 in dendritic cells mediated by TNF-α and IL-4, J. Leukoc. Biol. 66: 837-844.

F. Liao et al., 1999, CC-chemokine receptor 6 is expressed on diverse memory subsets of T cells and determines responsiveness to macrophage inflammatory protein 3α, J. Immunol. 162: 186-194.

R. Varona et al., 1998, Molecular cloning, functional characterization and mRNA expression analysis of the murine chemokine receptor CCR6 and its specific ligand MIP-3α, FEBS Lett. 440: 188-194.

A. Iwasaki y B. L. Kelsall, 2000, Localization of distinct Peyer's patch dendritic cell subsets and their recruitment by chemokines macrophage inflammatory protein (MIP)-3α, MIP-3β, and secondary lymphoid organ chemokine, J. Exp. Med. 191: 1.381-1.394.

J. J. Campbell et al., 1998, Chemokines and the arrest of lymphocytes rolling under flow conditions, Science 279: 381-384.

Y. Tanaka et al., 1999, Selective expression of liver and activation-regulated chemokine (LARC) in intestinal epithelium in mice and humans, Eur. J. Immunol. 29: 633-642.

A. S. Charbonnier et al., 1999, Macrophage inflammatory protein 3α is involved in the constitutive trafficking of epidermal Langerhans cells, J. Exp. Med. 190: 1.755-1.768.

B. Homey et al., 2000, Up-regulation of macrophage inflammatory protein-3∀/CCL20 and CC chemokine receptor 6 in psoriasis, J. Immunol., 164: 6.621-32.

M. C. Dieu-Nosjean et al., 2000, Macrophage inflammatory protein 3α is expressed at inflamed epithelial surfaces and is the most potent chemokine known in attracting Langerhans cell precursors, J. Exp. Med. 192: 705-717.

C. Nagler-Anderson, Man the barrier! Strategic defences in the intestinal mucosa, Nat. Rev. Immunol., 2001, 1: 59-67.

R. S. Blumberg, L. J. Saubermann y W. Strober, Animal models of mucosal inflammation and their relation to human inflammatory bowel disease, Curr. Opin. Immunol. 1999, 11: 648-656.

P. M. Murphy, M. Baggiolini, I. F. Charo, C. A. Hebert, R. Horuk, K. Matsushima, L. H. Miller, J. J. Oppenheim y C. A. Power, International Union of Pharmacology. XXII. Nomenclature for chemokine receptors. Pharmacol. Rev. 2000, 52: 145-176.

A. Zlotnik y O. Yoshie, Chemokines: a new classification system and their role in immunity, Immunity 2000,12: 121-127.

B. Moser y P. Loetscher, Lymphocyte traffic control by chemokines, Nat. Immunol. 2001, 2: 123-128.

K. M. Ansel y J. G. Cyster, Chemokines in lymphopoiesis and lymphoid organ development, Curr. Opin. Immunol. 2001, 13: 172-179.

F. Sallusto, C. R. Mackay y A. Lanzavecchia, The role of chemokine receptors in primary, effector, and memory immune responses, Annu. Rev. Immunol. 2000, 18: 593-620.

C. Gerard y B. J. Rollins, Chemokines and disease, Nat. Immunol. 2001, 2: 108-115.

L. Mazzucchelli, C. Hauser, K. Zgraggen, H. Wagner, M. Hess, J. A. Laissue y C. Mueller, Expression of interleukin-8 gene in inflammatory bowel disease is related to the histological grade of active inflammation, Am. J. Pathol. 1994, 144: 997-1.007.

M. C. Grimm, S. K. Elsbury, P. Pavli y W. F. Doe, Enhanced expression and production of monocyte chemoattractant protein-1 in inflammatory bowel disease mucosa, J. Leukoc. Biol. 1996. 59: 804-812.

S. Keates, A. C. Keates, E. Mizoguchi, A. Shan y C. P. Kelly, Enterocytes are the primary source of the chemokine ENA-78 in normal colon and ulcerative colitis, Am. J. Physiol. 1997. 273: G75-G82.

J. Wedemeyer, A. Lorentz, M. Goke, P. N. Meier, P. Flemming, C. A. Dahinden, M. P. Manns y S. C. Bischoff, Enhanced production of monocyte chemotactic protein 3 in inflammatory bowel disease mucosa, Gut 1999, 44: 629-635.

L. Mazzucchelli, C. Hauser, K. Zgraggen, H. E. Wagner, M. W. Hess, J. A. Laissue y C. Mueller, Differential in situ expression of the genes encoding the chemokines MCP-1 and RANTES in human inflammatory bowel disease, J. Pathol. 1996, 178: 201-206.

M. C. Dieu-Nosjean, B. Vanbervliet, A. Vicari, J. M. Bridon, E. Oldham, S. Aït-Yahia, F. Briere, A. Zlotnik, S. Lebecque y C. Caux, Selective recruitment of immature and mature dendritic cells by distinct chemokines expressed in different anatomic sites, J. Exp. Med. 1998, 188: 373-386.

A. Izadpanah, M. B. Dwinell, L. Eckmann, N. M. Varki y M. F. Kagnoff, Regulated MIP-3α/CCL20 production by human intestinal epithelium: mechanism for modulating mucosal immunity, Am. J. Physiol. Gastrointest. Liver Physiol. 2001, 280: G710-G719.

D. N. Cook, D. M. Prosser, R. Forster, J. Zhang, N. A. Kuklin, S. J. Abbondanzo, X. D. Niu, S. C. Chen, D. J. Manfra, M. T. Wiekowski, L. M. Sullivan, S. R. Smith, H. B. Greenberg, S. K. Narula, M. Lipp y S. A. Lira, CCR6 mediates dendritic cell localization, lymphocyte homeostasis, and immune responses in mucosal tissue, Immunity 2000, 12: 495-503.

R. Varona, R. Villares, L. Carramolino, I. I. Goya, A. A. Zaballos, J. Gutiérrez, M. Torres, A. C. Martinez y G. Márquez, CCR6-deficient mice have impaired leukocyte homeostasis and altered contact hypersensitivity and delayed-type hypersensitivity responses, J. Clin. Invest. 2001, 107: R37-R45.

V. T. Ho y R. J. Soiffer, The history and future of T-cell depletion as graft-versus-host disease prophylaxis for allogeneic hematopoietic stem cell transplantation, Blood 98: 3.192-3.204 (2001).

F. Sallusto, D. Lenig, R. Forster, M. Lipp y A. Lanzavecchia, Two subsets of memory T lymphocytes with distinct homing potentials and effector functions, Nature 401, 708-712 (1999).

J. H. Antin, Acute graft-versus-host disease: inflammation run amok?, J. Clin. Invest. 107: 1.497-1.498 (2001).

D. Snider y H. Liang, Early intestinal Th1 inflammation and mucosal T cell recruitment during acute graft-versus-host reaction, J. Immunol. 166, 5.991-5.999 (2001).

W. Krenger y J. L. Ferrara, Graft-versus-host disease and the Th1/Th2 paradigm, Immunol. Res. 15: 50-73 (1996).

W. D. Shlomchik et al., Prevention of graft versus host disease by inactivation of host antigen-presenting cells, Science 285, 412-415 (1999).

T. Teshima et al., Acute graft-versus-host disease does not require alloantigen expression on host epithelium, Nat. Med. 8, 575-581 (2002).

E. J. Kunkel y E. C. Butcher, Chemokines and the tissue-specific migration of lymphocytes, Immunity 16, 1-4 (2002).

E. Schutyser, S. Struyf y J. Van Damme, The CC chemokine CCL20 and its receptor CCR6, Cytokine Growth Factor Rev. 14, 409-426 (2003).

F. Liao et al., CC-chemokine receptor 6 is expressed on diverse memory subsets of T cells and determines responsiveness to macrophage inflammatory protein 3α, J. Immunol. 162: 186-194 (1999).

D. N. Cook et al., CCR6 mediates dendritic cell localization, lymphocyte homeostasis, and immune responses in mucosal tissue, Immunity 12, 495-503 (2000).

R. Varona et al., CCR6-deficient mice have impaired leukocyte homeostasis and altered contact hypersensitivity and delayed-type hypersensitivity responses, J. Clin. Invest. 107: R37-R45 (2001).

R. Varona, V. Cadenas, J. Flores, A. C. Martínez y G. Márquez, CCR6 has a non-redundant role in the development of inflammatory bowel disease, Eur. J. Immunol. 33: 2.937-2.946 (2003).

C. P. Larsen et al., 1990, Migration and maturation of Langerhans cells in skin transplants and explants, J. Exp. Med. 172: 1.483-1.493.

P. G. Andres, P. L. Beck, E. Mizoguchi, A. Mizoguchi, A. K. Bhan, T. Dawson, W. A. Kuziel, N. Maeda, R. R. MacDennott, D. K. Podolsky y H. C. Reinecker, Mice with a selective deletion of the CC chemokine receptors 5 or 2 are protected from dextran sodium sulfate-mediated colitis: lack of CC chemokine receptor 5 expression results in a NK1.1+ lymphocyte-associated Th2-type immune response in the intestine, J. Immunol. 2000, 164: 6.303-6.312.

H. Mashimo, D. C. Wu, D. K. Podolsky y M. C. Fishman, Impaired defense of intestinal mucosa in mice lacking intestinal trefoil factor, Science 1996, 274: 262-265.

M. F. Neurath, I. Fuss, B. L. Kelsall, E. Stuber y W. Strober, Antibodies to interleukin 12 abrogate established experimental colitis in mice, J. Exp. Med. 1995, 182: 1.281-1.290.

V. T. Ho y R. J. Soiffer, The history and future of T-cell depletion as graft-versus-host disease prophylaxis for allogeneic hematopoietic stem cell transplantation, Blood 98: 3.192-3.204 (2001).

F. Sallusto, D. Lenig, R. Forster, M. Lipp y A. Lanzavecchia, Two subsets of memory T lymphocytes with distinct homing potentials and effector functions, Nature 401, 708-712 (1999).

J. H. Antin, Acute graft-versus-host disease: inflammation run amok?, J. Clin. Invest. 107, 1.497-1.498 (2001).

D. Snider y H. Liang, Early intestinal Th1 inflammation and mucosal T cell recruitment during acute graft-versus-host reaction, J. Immunol. 166, 5.991-5.999 (2001).

W. Krenger y J. L. Ferrara, Graft-versus-host disease and the Th1/Th2 paradigm, Immunol. Res. 15, 50-73 (1996).

W. D. Shlomchik et al., Prevention of graft versus host disease by inactivation of host antigen-presenting cells, Science 285, 412-415 (1999).

T. Teshima et al., Acute graft-versus-host disease does not require alloantigen expression on host epithelium, Nat. Med. 8, 575-581 (2002).

E. J. Kunkel y E. C. Butcher, Chemokines and the tissue-specific migration of lymphocytes, Immunity 16, 1-4 (2002).

E. Schutyser, S. Struyf y J. Van Damme, The CC chemokine CCL20 and its receptor CCR6, Cytokine Growth Factor Rev. 14, 409-426 (2003).

F. Liao, et al., CC-chemokine receptor 6 is expressed on diverse memory subsets of cells and determines responsiveness to macrophage inflammatory protein 3α, J. Immunol. 162, 186-194 (1999).

D. N. Cook et al., CCR6 mediates dendritic cell localization, lymphocyte homeostasis, and immune responses in mucosal tissue, Immunity 12, 495-503 (2000).

R. Varona et al., CCR6-deficient mice have impaired leukocyte homeostasis and altered contact hypersensitivity and delayed-type hypersensitivity responses, J. Clin. Invest. 107, R37-R45 (2001).

R. Varona, V. Cadenas, J. Flores, A. C. Martinez y G. Márquez, CCR6 has a non-redundant role in the development of inflammatory bowel disease, Eur. J. Immunol. 33, 2.937-2.946 (2003).

W. Strober, I. J. Fuss y R. S. Blumberg, The immunology of mucosal models of inflammation, Annu. Rev. Immunol. 2002, 20: 495-549.

C. O. Elson, K. W. Beagley, A. T. Shannanov, K. Fujihashi, H. Kiyono, G. S. Tennyson, Y. Cong, C. A. Black, B. W. Ridwan y J. R. McGhee, Hapten-induced model of murine inflammatory bowel disease: mucosa immune responses and protection by tolerance, J. Immunol. 1996, 157: 2.174-2.185.

R. Varona et al., Molecular cloning, functional characterization and mRNA expression analysis of the murine chemokine receptor CCR6 and its specific ligand MIP-3α, 440 FEBS Lett. 188 (1998).

M. Torres, 1.998, The use of embryonic stem cells for the genetic manipulation of the mouse, 36 Curr. Top. Dev. Biol. 99 (1998).

## Claims

1. A mouse cell line designated CCR6KOES192, deposited with the DSM under accession number DSM ACC2636.

2. A mammalian cell having a deletion in at least one CCR6 allele characteristic of the mouse cell line designated CCR6KOES192, deposited with the DSM under accession number DSM ACC2636.

3. A mouse, or part thereof, comprising cells grown from the mammalian cell of Claim 1.

4. A mouse, or part thereof, comprising cells grown from the mammalian cell of Claim 2.

5. A transgenic, non-human, knockout animal whose genome comprises an introduced null mutation in at least one CCR6 allele, wherein said mutation has the characteristics of the cell line designated CCR6KOES192, deposited with the DSM under accession number DSM ACC2636.

6. The knockout animal of claim 5, wherein said animal is a rodent.

7. The knockout rodent of claim 6, wherein said rodent is a mouse.

8. The knockout mouse of claim 7 whose genome comprises an introduced null mutation in both CCR6 alleles.

9. A method of making a transgenic knockout mouse having a disrupted CCR6 gene comprising:
deleting the first exon of the CCR6 gene including the initiation codon by homologous recombination in mouse embryonic stem cells;
aggregating said embryonic stem cells with morulae and allowing the cells to reach the blastocyst stage *in vitro;*
transplanting said blastocyst into a pseudopregnant mouse;
allowing said blastocyst to develop into a chimeric mouse;
breeding said chimeric mouse to produce offspring; and
screening said offspring to identify a homozygous transgenic knockout mouse whose genome comprises deletion of the first exon of both alleles of the CCR6 gene.

10. The method of making a transgenic knockout mouse having a disrupted CCR6 gene, according to claim 9, wherein said embryonic stem cells comprise cells grown from a CCR6KOES192 cell line deposited as Accession Number DSM ACC2636.

11. A method for screening for a test compound that modulates CCR6 activity, said method comprising:
exposing CCR6 to a test *compound in vitro;*
assaying for binding of the test compound to CCR6; and
assaying whether the test compound modulates CCR6 activity.

12. The method for screening for a test compound that modulates CCR6 activity, according to Claim 11, wherein said CCR6 is expressed on a cell surface.

13. The method for screening for a test compound that modulates CCR6 activity, according to Claim 12, wherein said cell is selected from the group consisting of mouse and human transfectant cells, CCR6-expressing primary cells, and CCR6-expressing cell lines.

14. A method to assay for a test compound that moderates CCR6-dependent GvHD comprising:
providing CD4⁺ T cells from a CCR6 WT mouse;
providing a CCR6 WT, MHC(II)-mismatched or MHC-matched, mhc-mismatched mouse;
providing a test compound;
transferring said T cells to said WT mouse in the presence and absence of said test compound; and
comparing a GvHD response in the presence and absence of said test compound.

15. The method to assay for a test compound that moderates CCR6-dependent GvHD, according to claim 14, wherein said GvHD response is selected from the group consisting of % survival at inflection day post transfer, recipient weight at inflection day, ear inflammation, skin lesions, diarrhea, spleen index, and CD4⁺ T cell infiltration into the GvHD-affected tissues.

16. A method to assay for a test compound that can revert the lack of regulatory function of CCR6-deficient CD4⁺CD45RB^{low} regulatory T cells comprising:
providing CD4⁺ T cells from a CCR6 WT mouse;
providing CD4⁺CD45RB^{low} regulatory T cells from a CCR6-deficient mouse;
providing a CCR6 WT, MHC(II)-mismatched or MHC-matched, mhc-mismatched mouse;
providing a test compound;
cotransferring said T cells to said WT mouse in the presence and absence of said test compound; and
comparing a GvHD response in the presence and absence of said test compound.

17. A method to assay for a test compound that moderates CCR6-dependent, inflammatory bowel disease comprising:
providing a C57BL/6J WT mouse;
rendering said mice as DSS-type IBD models;
administering a test compound to said mice; and
comparing an IBD response of said WT mouse in the presence and
absence of said test compound.

18. The method to assay for a test compound that moderates CCR6-dependent, inflammatory bowel disease, according to claim 17, wherein said IBD response is selected from the group consisting of intestinal inflammation and colonic mucosa Th1 and Th2 cytokine mRNA levels.

19. A method to assay for a test compound that moderates CCR6-dependent, inflammatory bowel disease comprising:
providing a C57BL/6J WT mouse;
administering trinitrobenzenesulfonic acid to said mice;
administering a test compound to said mice; and
comparing an IBD response of said WT mouse in the presence and
absence of said test compound.

20. The method to assay for a test compound that moderates CCR6-dependent, inflammatory bowel disease, according to claim 19, wherein said IBD response is selected from the group consisting of intestinal inflammation and colonic mucosa Th1 and Th2 cytokine mRNA levels.

21. A method to assay for a test compound that moderates CCR6-dependent, contact hypersensitivity cutaneous inflammation comprising:
providing a C57BL/6J WT mouse;
sensitizing said mice with DNFB;
challenging said mice with DNFB;
administering a test compound to said mice; and
comparing a contact hypersensitivity cutaneous inflammation response of said WT mouse in the presence and absence of said test compound.

22. The method to assay for a test compound that moderates CCR6-dependent, contact hypersensitivity inflammation according to claim 21, wherein said contact hypersensitivity inflammation response is ear inflammation.

23. A method to assay for a test compound that moderates a CCR6-dependent delayed-type hypersensitivity inflammatory response comprising:
providing a CCR6 WT mouse;
providing a test compound;
injecting said mice with allogeneic splenocytes;
challenging said mice with allogeneic splenocytes; and
comparing a footpad swelling in the presence and absence of said test compound.

24. The method to assay for a test compound that moderates a CCR6-dependent delayed-type hypersensitivity inflammatory response, according to claim 23, wherein said allogeneic splenocytes are BALB/c.

25. The method to assay for a test compound that moderates a CCR6-dependent delayed-type hypersensitivity inflammatory response, according to claim 23, wherein said test compound is provided systemically.
